# EUROPEAN PATENT APPLICATION

(11) **EP 2 008 529 A1**
(43) Date of publication of application: **31.12.2008**
(21) Application number: 07738219.0
(22) Date of filing: 09.03.2007
(51) Int. Cl.: A23G 3/34, A61K 38/46, A61P 1/02, A23L 1/30

(54) **ENZYME-CONTAINING CANDY**

(30) Priority: 10.03.2006 JP 2006066321
(71) Applicant: Ezaki Glico Co., Ltd., Osaka-shi, Osaka 555-8502 (JP)
(72) Inventor: YOSHIMATSU, Daisuke, Amagasaki-shi, Hyogo 661-0014 (JP); IOKA, Toshiyuki, Nishinomiya-shi, Hyogo 663-8113 (JP)
(74) Representative: UEXKÜLL & STOLBERG
(86) International application number: PCT/JP2007/054734
(87) International publication number: WO 2007/105661

(57) **Abstract**

It is intended to improve a method of adding a non-thermostable enzyme to a candy base material. In the method of the invention, a non-thermostable enzyme is added to a candy base material, and the material is stirred so that a substantially uniform mixed dough-like mixture is obtained within 5 minutes from the addition to obtain the mixed dough-like mixture. Then, the mixed dough-like mixture is cooled on a cooling plate and the mixed dough-like mixture is shaped. Preferably, amain component of the candy base material is selected from the group consisting of reduced palatinose, maltitol, reduced starch syrup, and xylitol.

## Description

### TECHNICAL FIELD

The present invention relates to an enzyme-containing candy.

### BACKGROUND ART

Enzymes have various activities. It is expected that by incorporating an enzyme in a candy, the activity of the enzyme is exerted to obtain various useful effects.

The candy is generally produced by melting a saccharide and the like. Heating at a high temperature such as about 150° C or higher is required for melting a saccharide. If the enzyme is added to such a high-temperature melt, the enzyme is inactivated and the enzyme activity is hardly obtained in the resultant candy.

Furthermore, since candy stays in the oral cavity for a long time, it is desirable that a saccharide (such as sugar) which causes carcinogenicity is not blended and a noncarcinogenic saccharide is blended. A candy that does not contain sugar is generally referred as a sugarless candy. On the other hand, a candy containing sugar is referred as a sugar candy. In order to produce a sugarless candy containing an enzyme, in view of ease of mixing , it is necessary to add the enzyme at a higher temperature than that of a sugar candy by about 30° C. That is, in the case of a sugar candy, the enzyme may be added in the vicinity of 100° C, but in the case of the production of a sugarless candy, if the temperature of the mixture is not raised to about 130° C, the mixture becomes uneven and is not suitable for industrial production. In the case of a general candy not containing an enzyme, if the mixture is at a high temperature of about 130° C, there is no problem except for productivity lowering. However, enzyme is generally weak for heat and therefore is not thought to be resistant to the temperature of about 130°C. Furthermore, also in the case of the sugar candy, even if the enzyme is added in the vicinity of 100° C, by a conventional method, the enzyme would be inactivated and the candy having the enzyme activity cannot be obtained.

Various methods for obtaining a candy having an enzyme activity have been investigated. Patent Document 1 (Japanese Laid-open Patent Publication No. 2-86731) describes a method for producing an enzyme-containing hard candy, which comprises producing a hard candy not containing an enzyme, crushing the hard candy, granulating it to be a 100-mesh path, mixing the granulated matter with an enzyme, compressing it to obtain a tablet, and then heating it to 100 to 140° C to be melted while being pressurized by an inactive gas. The method described in Patent Document 1 is specifically characterized by mixing a saccharide raw material and water, then boiling the mixture until the water content becomes 3.0% by weight or less, crushing the hard candy obtained by cooling and solidifying the mixture, granulating the hard candy to have a granularity that is capable of passing through a JIS standard sieve 100 mesh, adding and mixing about one parts by weight of one or two or more kinds of enzyme selected form the group consisting of cellulase, amylase, lipase, protease, lysozyme, and dextranase to 100 parts by weight of the granulated hard candy, then shaping it, followed by housing it in a container that can be pressurized, partially melting it by heating it with the container under increased pressure with an inactive gas, cooling the obtained partially melted matter with the container to solidify the matter, and then releasing the pressuring state in the container obtained by an inactive gas. In the method described in Patent Document 1, the hard candy is granulated to have a granularity that is capable of passing through a JIS standard sieve 100 mesh, but the hygroscopicity of the candy having such granularity which is obtained by crushing is very high and the candy is easy to be consolidated even at low humidity. Therefore, it is thought that the method can be carried out at a test production level, but it is actually impossible to mass-produce the candy in a factory. Furthermore, the sieve of the 100 mesh have very fine grid and therefore it is predicted that clogging happens in a short period and the method is not practical. For making the powder into tablets, it is necessary that the powder has appropriate flowability, and it is not practical to compress the crushed candy that is easy to absorb moisture and to be consolidated, into a tablet. Furthermore, in this method, it is necessary that the candy is housed in the container that can be pressurized, and heated to 100 to 140° C while being pressurized with an inactive gas. To performing this step, a special device is required, and since such a device is expensive, the production cost becomes high, and also there is disadvantage that the equipment is too expensive for producing the candy whose unit price is relatively low. In the section "prior art" of Patent Document 1, it is described that in a method of directly adding and blending an enzyme into a saccharide raw material, the enzyme is inactivated by thermal denaturation and functional characteristics of the enzyme are completely lost. This indicates that it is not preferable to add the enzyme directly to the saccharide raw material.

In Patent Document 2 (Japanese Laid-open Patent Publication No. 2001-086952), flavor-improving agent and intoxication-preventing food using the agent are described. In paragraph 0011 of Patent Document 2, foods such as chewing gum, candy, and jelly containing alcohol dehydrogenase (ADH) derived from a plant are described. In paragraph 0031 of Patent Document 2, chewing gum, fondant, soft candy, tablet candy, jelly, gummy, and the like are described as low-temperature heated or non-heated foods or low-moisture foods. In paragraph 0032 of Patent Document 2, as a method for producing such a food, it is described that, by mixing ADH derived from a plant with various food raw materials, using the obtained mixture according to a conventionally known method for producing various foods, intoxication-preventing food is obtained. However, in order to obtain a fondant, soft candy, jelly, gummy, and the like, heating at a high temperature of more than about 120° C are necessary, and therefore, if an enzyme is added directly to the materials thereof, the enzyme becomes inactivated. In paragraph 0035 of Patent Document 2, it is described that for high-temperature heated foods such as hard candy, in order to prevent inactivation of the enzyme, it is preferable that the enzyme is not directly mixed in the dough-like mixture of a candy but an outer surface of the preliminarily produced food is dredged or coated with the enzyme. In Example 10 of Patent Document 2, it is described that after producing the hard candy, a flavor-improving agent is sprayed on the outer surface thereof, and then, an ADH-containing powder saccharide is dredged to form a coating layer, and thereby, the hard candy with the coating layer is obtained.

Patent Document 3 (Japanese Laid-open Patent Publication No. 2001-172151) describes a composition for an oral cavity characterized in that it comprises β-1,4-glucanase. The composition for an oral cavity described in Patent Document 3 is intended removal of dirt of teeth attached or deposited on the tooth surface. The compositions for an oral cavity described in Patent Document 3 are dentifrice, mouth wash, troche, chewing gum, and the like, and none of the production process of these compositions require high temperature. In Patent Document, a candy is not described at all.

Patent Document 4 (Japanese Laid-open Patent Publication No. 2003-219809) describes a sugar-coated product characterized by having a sugarcoating layer containing an enzyme. Examples of sugarcoated products described in Patent Document 4 include medical products such as tablet and troche as well as confectionaries such as chewing gum, tablet candies, gummy, capsule, and candy. An object of Patent Document 4 is to provide a product taking short time for enzyme-releasing. Patent Document 4 sees it as a problem that with respect to the conventional technique, if an enzyme is blended inside the product, releasing the enzyme takes long period, and suggested that blending of the enzyme inside the product is not preferable.

Patent Document 5 (Japanese Laid-open Patent Publication No. 2003-9784) describes methods for eliminating the odor and improving the flavor of foods. One of purposes of the foods and drinks described in Patent Document 5 is to suppress the odor of a mouth of a human. Patent Document 5 describes foods and drinks having odor-eliminating effects containing a treated plant-tissue having oxyreductase activities. Examples of the foods and drinks include gum, tablet, candy, gummy, and chocolate. Paragraph 0018 of Patent Document 5 describes that when processing the product, it is desirable to add a treated plant-tissue under the condition of short time exposure to a relatively high temperature in the final step of the processing, to treat the product. It is a well known fact that an enzyme is weak for heat and this is natural. However, there are no detailed descriptions as to what heat temperature should be used, how long heat time should be used, and what method of dispersing the enzyme should be used at the final stage of processing the product in order to achieve the addition of the enzyme without inactivation, and thus, it is necessary to specifically investigate each of the foods and drinks. The treated plant-tissues described in Patent Document 5 are pulp produced from a crushed liquid of a plant tissue, an insoluble fraction produced from a squeeze liquid of a plant tissue, sieved pulp produced from a squeeze liquid of a plant tissue, or dried matters thereof. The treated plant-tissue is water-insoluble and therefore if it is added in quite small amount, the surface of candy becomes rough and the feeling of the mouth becomes bad. Even in the case where roughness is preferable in purpose of removing tongue coating, if an amount of an enzyme required for tongue coating removal is added, the surface becomes to be rough beyond necessity, and the palatability significantly becomes low.

Patent Document 6 (Japanese Laid-open Patent Publication No. 2005-281230) describes a solid composition for an oral cavity. The composition for an oral cavity is composed of a water-soluble solid substrate blended with an insoluble natural plant fiber. The composition for oral cavity aims for reduction of halitosis by physical removal of tongue coating with fine projections formed by the plant fiber on the substrate surface. In Patent Document 6, there is no description concerning the blend of an enzyme.

Patent Document 7 (International Publication No. WO 2003/090704 pamphlet) describes a food having an action of removing tongue coating. Patent Document 7 is a publication of an application by the present applicant. Patent Document 7 describes a candy as the food. Patent Document 7 describes, with respect to addition of a compound or a composition having an action removing halitosis or an action of removing tongue coating to a candy, that after bolding a material of the candy according to a general method, during cooling the boiled material, the compound or the composition is added at the time of preferably about 60° C or lower, more preferably about 50° C or lower, and further more preferably about 40° C or lower. However, if the material of the candy is added at the time of about 60° C or lower in cooling after boiling the material of the candy, the candy is solidified before the material of the candy and the compound or the composition are uniformly mixed, and therefore, the candy containing the compound or the composition and the candy not containing it are produced. Therefore, the disposal ratio becomes high and the production cost becomes high. Therefore, there is a problem that the industrial production is difficult.
[Patent Document 1] Japanese Laid-open Patent Publication No. 2-86731 (pp 1-4)
[Patent Document 2] Japanese Laid-open Patent Publication No. 2001-086952 (pp 1-6)
[Patent Document 3] Japanese Laid-open Patent Publication No. 2001-172151 (pp 1-13)
[Patent Document 4] Japanese Laid-open Patent Publication No. 2003-219809 (pp 1-5)
[Patent Document 5] Japanese Laid-open Patent Publication No. 2003-9784 (pp 1-18)
[Patent Document 6] Japanese Laid-open Patent Publication No. 2005-281230 (pp 1-13)
[Patent Document 7] International Publication No. WO 2003/090704 pamphlet (pp 1-70)

### DISCLOSURE OF THE INVENTION

### PROBLEMS TO BE SOLVED BY THE INVENTION

The present invention is intended to solve the above-mentioned problems, and an object of the present invention is to provide a method for producing a candy having enzyme activity at a low cost.

### MEANS TO SOLVE THE PROBLEMS

Conventionally, it has been known that preservability of an enzyme is low in an aqueous solution but the preservability is improved when the enzyme is dried. However, it has not been known that heat stability of the enzyme is improved by drying (except for an oxyreductase in the treated plant-tissue, which is described later). Therefore, it has not been possible to efficiently produce candies having an enzyme activity.

As a result of diligent studies, the present inventors have found that by improving the method for adding a non-thermostable enzyme to a candy base material, the candy having enzyme activities is efficiently produced. Based on this finding, the present inventors completed the present invention.

The method of the invention is a method for producing an enzyme-containing candy, in which
a candy base material is heated to a dissolution temperature,
the candy base material is heated from the dissolution temperature to a moisture evaporation temperature,
the candy base material is cooled from the moisture evaporation temperature to a first cooling temperature, then
the candy base material is cooled from the first cooling temperature to a second cooling temperature, then
the candy base material is cooled from the second cooling temperature to a third cooling temperature, and then
the candy base material is cooled from the third cooling temperature to a fourth cooling temperature,
wherein there is no substantial increase of the temperature of the material from the first cooling temperature to the fourth cooling temperature,
the dissolution temperature is 100° C or higher and 143° C or lower,
the moisture evaporation temperature is 100° C or higher and 180° C or lower,
the first cooling temperature is 100° C or higher and 135° C or lower,
the second cooling temperature is 80° C or higher and 122° C or lower,
the third cooling temperature is 50° C or higher and 95° C or lower, and
the fourth cooling temperature is less than 50°C, and wherein
the method comprising the steps of:
(a) adding a non-thermostable enzyme to the candy base material, stirring the material so as to obtain a substantially uniform mixed dough-like mixture within 5 minutes from the addition, and thereby obtaining the mixed dough-like mixture;
(b) cooling the mixed dough-like mixture on a cooling plate; and
(c) shaping the mixed dough-like mixture;
wherein step (a) is performed while the temperature of the candy base material is between the first cooling temperature and the second cooling temperature;
the step (b) is performed while the temperature of the candy base material is between the second cooling temperature and the third cooling temperature;
the step (c) is performed while the temperature of the candy base material is between the third cooling temperature and the fourth cooling temperature;
the period of time from the addition of the non-thermostable enzyme to the candy base material until reaching the second cooling temperature is 5 minutes or less;
the period of time from the second cooling temperature to reaching the third cooling temperature is 5 minutes or less; and
the period of time from the third cooling temperature to reaching the fourth cooling temperature is 20 minutes or less.

In a specific embodiment, especially in the case of a sugarless candy, the method of the invention is a method for producing an enzyme-containing candy, in which
a candy base material is heated to a dissolution temperature,
the candy base material is heated from the dissolution temperature to a moisture evaporation temperature,
the candy base material is cooled from the moisture evaporation temperature to a first cooling temperature, then
the candy base material is cooled from the first cooling temperature to a second cooling temperature, then
the candy base material is cooled from the second cooling temperature to a third cooling temperature, and then
the candy base material is cooled from the third cooling temperature to a fourth cooling temperature,
wherein there is no substantial increase of the temperature of the material from the first cooling temperature to the fourth cooling temperature,
the dissolution temperature is from 135° C to 143° C,
the moisture evaporation temperature is from 143°C to 180°C,
the first cooling temperature is from 125°C to 135°C,
the second cooling temperature is from 115° C to 122° C,
the third cooling temperature is from 80° C to 95° C, and
the fourth cooling temperature is from room temperature to 50° C, and wherein
the method comprising the steps of:
(a) adding a non-thermostable enzyme to the candy base material, stirring the material so as to obtain a substantially uniform mixed dough-like mixture within 5 minutes from the addition, and thereby obtaining the mixed dough-like mixture;
(b) cooling the mixed dough-like mixture on a cooling plate; and
(c) shaping the mixed dough-like mixture;
wherein step (a) is performed while the temperature of the candy base material is between the first cooling temperature and the second cooling temperature;
the step (b) is performed while the temperature of the candy base material is between the second cooling temperature and the third cooling temperature;
the step (c) is performed while the temperature of the candy base material is between the third cooling temperature and the fourth cooling temperature;
the period of time from the addition of the non-thermostable enzyme to the candy base material until reaching the second cooling temperature is 5 minutes or less;
the period of time from the second cooling temperature to reaching the third cooling temperature is 5 minutes or less; and
the period of time from the third cooling temperature to reaching the fourth cooling temperature is 20 minutes or less.

In both of the aforementioned methods, the following embodiments are preferable:
In one embodiment, a main component of the aforementioned candy base material is a saccharide.

In one embodiment, the aforementioned main component is a non-cariogenic saccharide.

In one embodiment, the aforementioned main component is selected from the group consisting of reduced palatinose, maltitol, reduced starch syrup, and xylitol.

In one embodiment, the aforementioned main component is reduced palatinose.

In one embodiment, the aforementioned candy base material does not substantially contain sugar.

In one embodiment, at the temperature is 80° C or lower the remaining activity is 20% or less after the aforementioned non-thermostable enzyme is heated for 30 minutes in a 20 mM Tris buffer (pH 7.0).

In one embodiment, the aforementioned non-thermostable enzyme is selected from the group consisting of a transferase, hydrolase, lyase, isomerase, and ligase.

In one embodiment, the aforementioned non-thermostable enzyme is selected from the group consisting of a protease, lipase, amylase, and dextranase.

In one embodiment, the aforementioned non-thermostable enzyme is a cysteine protease or serine protease.

In one embodiment, the aforementioned non-thermostable enzyme is a papain, bromelain, or actinidine.

In one embodiment, the aforementioned non-thermostable enzyme is added in a powder state.

In one embodiment, the weight of the aforementioned candy base material in step (a) is from 20 to 30 kg.

In one embodiment, the weight of the aforementioned non-thermostable enzyme in step (a) is from 0.2 to 1 kg.

In one embodiment, the aforementioned non-thermostable enzyme is substantially uniformly dispersed in the aforementioned enzyme-containing candy.

In one embodiment, the enzyme content of the aforementioned enzyme-containing candy is from 0.1 to 3.0% by weight.

In one embodiment, the aforementioned enzyme-containing candy is a candy for removing tongue coating, the aforementioned enzyme is a protease, and the aforementioned candy base material contains an insoluble powder.

In one embodiment, the aforementioned enzyme-containing candy is a candy for removing tongue coating, and the aforementioned enzyme is a protease, and wherein the method further comprises step (d) of kneading bubble in the mixed dough-like mixture, and step (d) is performed while the temperature of the candy base material is from the second cooling temperature to the third cooling temperature after step (b).

### EFFECT OF THE INVENTION

By the present invention, a candy having an enzyme activity can be produced at a low cost.

### BRIEF DESCRIPTION OF THE DRAWINGS

[Fig. 1] Fig. 1 is a view schematically showing an evaluation study design used in Evaluation Example 1.
[Fig. 2] Fig. 2 is a judgmental rating reference diagram for the amount of tongue coating.
[Fig. 3] Fig. 3 is a graph showing a rate of decrease (%) of the tongue coating score.
[Fig. 4] Fig. 4 is a graph showing a rate of change (%) of hydrogen sulfide.

### BEST MODE FOR CARRYING OUT THE INVENTION

Hereinafter, the present invention will be explained in detail.

The term "candy" in the present invention refers to a solid food in which the main raw material is saccharide or sugar alcohol and which is produced by a method including a step of boiling the saccharide or the sugar alcohol and water. The candy is categorized into a soft candy and a hard candy. Hardness of the candy is affected by the material of the candy and the boiling temperature at endpoint of heating.

Examples of the hard candy includes drop, butterscotch, peanut brittle, and bekko candy. Regarding an example of the case of mainly using sugar and starch syrup as the saccharide, a typical boiling temperature at endpoint of heating for producing drop is about 145° C. Regarding an example of the case of mainly using sugar and starch syrup as the saccharide, a typical boiling temperature at endpoint of heating for producing butterscotch is about 145°C, and the butterscotch contains about 4 to 6% by weight of butter and the like. Regarding an example of the case of mainly using sugar and starch syrup as the saccharide, a typical boiling temperature at endpoint of heating for producing peanut brittle is about 146° C, and the peanut brittle contains peanut, baking soda, and the like. Regarding an example of the case of mainly using sugar and starch syrup as the saccharide, a typical boiling temperature at endpoint of heating for producing bekko candy is about 150 to 160° C, a typical sugar to starch syrup ratio in solid content is sugar 40-60 : starch syrup 40-60, and they contain a milk product, oil, fat, and the like.

The candy of the present invention is preferably a hard candy, and more preferably, a drop.

### (1. Candy material)

The candy material includes a candy base material, a non-thermostable enzyme, and a minor material. In the present specification, the term "candy base material" refers to water and a material to be mixed and boiled with water to reduce, or a material which originally contains water and will be boiled until reduced. Even when the material is not mixed with water, a water containing material to be boiled to reduce, such as 70% maltitol syrup, is also included in the scope of the candy base material. In the present specification, the term "minor material" refers to a material that is added after almost all of water of the candy base material is evaporated by boiling, except for the non-thermostable enzyme.

### (1.1 Non-thermostable Enzyme)

The term "non-thermostable enzyme" in the present specification refers to an enzyme for which at the temperature is 80° C or lower the remaining activity is 20% or less after the enzyme is heated for 30 minutes in a 20 mM Tris buffer (pH 7.0). This does not mean that the enzyme used in the present invention has thermostability that is high out of common sense, but it means that the enzyme has a general extent of heat instability. Although the enzyme used in the present invention does not have extremely high thermostability, the present invention exerts significant effect that the enzyme can be blended in the candy requiring boiling at a high temperature. The temperature at which the remaining activity is 20% or less after the enzyme is heated for 30 minutes in a 20 mM Tris buffer (pH 7.0) is preferably about 40° C or higher, more preferably about 45° C or higher, further preferably about 50°C or higher. The temperature at which the remaining activity is 20% or less after the enzyme is heated for 30 minutes in a 20 mM Tris buffer (pH 7.0) may be about 75° C or lower, about 70° C or lower, or about 65° C or lower in one embodiment.

The temperature at which the remaining activity is 20% or less after the enzyme is heated for 30 minutes in a 20 mM Tris buffer (pH 7.0) is 78° C for papain. In the present invention, the enzyme having the same extent of thermostability as papain can be preferably used. The "same extent" means that the temperature for the enzyme is in the range of ±5°C of the temperature for papain. Examples of the enzyme having the same extent of thermostability as papain include bromelain and actinidine.

The enzyme used in the method of the present invention preferably has an activity at about 37°C.

The non- thermostable enzyme is preferably selected from the group consisting of transferase, hydrolase, lyase, isomerase, and ligase (also referred to as synthetase), and more preferably selected from the group consisting of protease, lipase, amylase, dextranase, and oxyreductase, and further more preferably selected from the group consisting of protease, lipase, amylase, and dextranase, and further preferably protease, and further more preferably selected from the group consisting of cysteine protease and serine protease, and particularly preferably cysteine protease in the papain family, and most preferably selected from the group consisting of papain, bromelain, and actinidine.

A protease (preferably selected from the group consisting of cysteine protease and serine protease, and more preferably selected form cysteine protease in the papain family, and most preferably selected from the group consisting of papain, bromelain, and actinidine) is useful for degrading and removing tongue coating and particularly useful if the protease is blended in the candy for removing tongue coating.

A lipase is useful for degrading oil components. In particular, if a lipase is blended in a candy which is taken after meal, the lipase can achieve an effect that oil components in an oral cavity are degraded and refreshed feeling is obtained.

An amylase and a dextranase are useful for degrading dental plaque, and when they are blended in a dental candy, the effect degrading dental plaque in an oral cavity can be exerted.

An oxyreductase is useful for degrading various bad-smelling substances, and particularly, when the enzyme is blended in the candy which is taken after meal, the effect degrading garlic odor, fishy odor, or the like can be exerted.

The non- thermostable enzyme is preferably added in a powder state. This is because in a powder state, the enzyme has high thermostability.

The non- thermostable enzyme is preferablyapurified. A purity of the non- thermostable enzyme is preferably about 50% by weight or more, more preferably about 60% by weight or more, further preferably about 70% by weight or more, and further more preferably about 80% by weight or more, particularly preferably about 90% by weight or more, and most preferably about 95% by weight or more. The purity of the non- thermostable enzyme is generally a purity of 100% by weight or less.

The amount to be used of the non- thermostable enzyme can vary depending on a type of the enzyme, but with respect to 100 parts byweight of the candy base material after moisture evaporation, the amount of the enzyme to be used is preferably about 0.1 parts by weight or more, more preferably about 0.2 parts by weight or more, further preferably about 0.5 parts by weight or more, most preferably about 1.0 parts by weight or more. With respect to 100 parts by weight of the candy base material after moisture evaporation, the amount to be used of the non- thermostable enzyme is preferably about 5.0 parts by weight or less, more preferably about 4.0 parts by weight or less, further preferably about 3.0 parts by weight or less, most preferably about 2.5 parts by weight or less. If the amount of the enzyme to be used is too small, the effect by the enzyme addition is occasionally not obtained. If the amount of the enzyme to be used is too large, the obtained effect is occasionally saturated and the cost-effectiveness occasionally become worsen. In the case that the flavor of the enzyme is strong (for example, in the case of an oxyreductase derived from a vegetable), if the amount of the oxyreductase to be used is too large, the flavor of the resultant candy occasionally become worsen.

For example, when protease is used as the non-thermostable enzyme, with respect to 100 parts by weight of the candy base material after moisture evaporation, the amount of protease to be used is preferably about 0.1 parts by weight or more, more preferably about 0.2 parts by weight or more, further preferably about 0.5 parts by weight or more, and most preferably about 1.0 parts by weight or more. With respect to 100 parts by weight of the candy base material after moisture evaporation, the amount of protease to be used is preferably about 5.0 parts by weight or less, more preferably about 4.0 parts by weight or less, further preferably about 3.0 parts by weight or less, and most preferably about 2.5 parts by weight or less. If the amount of protease to be used is too small, the effect by the enzyme addition is occasionally not obtained. If the amount of protease to be used is too large, the obtained effect may be saturated and the cost-effectiveness occasionally become worsen. If the amount of protease to be used is too large, stimulation to the oral cavity is occasionally too strong to result in a problem.

For example, when lipase is used as the non-thermostable enzyme, the amount of lipase to be used can vary depending on a type of the enzyme, but with respect to 100 parts byweight of the candybasematerial aftermoisture evaporation, the amount of lipase to be used is preferably about 0.1 parts by weight or more, more preferably about 0.2 parts by weight or more, further preferably about 0.5 parts by weight or more, and most preferably about 1. 0 parts by weight or more. With respect to 100 parts by weight of the candy base material after moisture evaporation, the amount of lipase to be used is preferably about 5.0 parts by weight or less, more preferably about 4.0 parts by weight or less, further preferably about 3.0 parts by weight or less, and most preferably about 2.5 parts by weight or less. If the amount of lipase to be used is too small, the effect by the enzyme addition is occasionally not obtained. If the amount of lipase to be used is too large, the obtained effect may be saturated and the cost-effectiveness occasionally become worsen.

For example, when amylase is used as the non-thermostable enzyme, the amount of amylase to be used can vary depending on a type of the enzyme, but with respect to 100 parts byweight of the candy base material aftermoisture evaporation, the amount of amylase to be used is preferably about 0.1 parts by weight or more, more preferably about 0.2 parts by weight or more, further preferably about 0.5 parts by weight or more, and most preferably about 1.0 parts by weight or more. With respect to 100 parts by weight of the candy base material after moisture evaporation, the amount of amylase to be used is preferably about 5.0 parts by weight or less, more preferably about 4.0 parts by weight or less, further preferably about 3.0 parts by weight or less, and most preferably about 2.5 parts by weight or less. If the amount of amylase to be used is too small, the effect by the enzyme addition is occasionally not obtained. If the amount of amylase to be used is too large, the obtained effect is occasionally saturated and the cost-effectiveness occasionally become worsen.

For example, when dextranase is used as the non-thermostable enzyme, the amount of dextranase to be used can vary depending on a type of the enzyme, but with respect to 100 parts byweight of the candybasematerial aftermoisture evaporation, the amount of dextranase to be used is preferably about 0.1 parts by weight or more, more preferably about 0.2 parts by weight or more, further preferably about 0.5 parts by weight or more, and most preferably about 1.0 parts by weight or more. With respect to 100 parts by weight of the candy base material after moisture evaporation, the amount of dextranase to be used is preferably about 5. 0 parts by weight or less, more preferably about 4.0 parts by weight or less, further preferably about 3.0 parts by weight or less, and most preferably about 2.5 parts by weight or less. If the amount of dextranase to be used is too small, the effect by the enzyme addition is occasionally not obtained. If the amount of dextranase to be used is too large, the obtained effect is occasionally saturated and the cost-effectiveness occasionally become worsen.

For example, when oxyreductase is used as the non-thermostable enzyme, the amount of oxyreductase to be used can vary depending on a type of the enzyme, but with respect to 100 parts byweight of the candybasematerial after moisture evaporation, the amount of oxyreductase to be used is preferably about 0.1 parts by weight or more, more preferably about 0.2 parts by weight or more, further preferably about 0.5 parts by weight or more, and most preferably about 1.0 parts by weight or more. With respect to 100 parts by weight of the candy base material after moisture evaporation, the amount of oxyreductase to be used is preferably about 5.0 parts by weight or less, more preferably about 4.0 parts by weight or less, further preferably about 3.0 parts by weight or less, and most preferably about 2.5 parts by weight or less. If the amount of oxyreductase to be used is too small, the effect by the enzyme addition is occasionally not obtained. If the amount of oxyreductase to be used is too large, the obtained effect is occasionally saturated and the cost-effectiveness occasionally become worsen. Moreover, because the flavor is strong in the case that an oxyreductase is derived from vegetable, if the amount of the vegetable-derived oxyreductase is too large, the flavor of the resultant candy occasionally become worsen.

### (1.2 Candy base material)

As the candy base material, a saccharide is mainly used. The "saccharide" in the present specification refers to as a compound composed of carbon, hydrogen, and oxygen. The saccharide includes saccharide and carbohydrate. The saccharide is preferably a compound having sweet taste. The saccharide is preferably selected from the group consisting of monosaccharide, disaccharide, oligosaccharide, sugar alcohol, starch syrup, decomposed starch, and water-soluble dietary fiber, more preferably selected from the group consisting of monosaccharide, disaccharide, oligosaccharide, and sugar alcohol. As the saccharide, any saccharide that is commercially available in the art can be used. In the candy of the present invention, two or more kinds of saccharide can be used in combinations. The saccharide used in the candy of the present invention is preferablywater-soluble. If two ormore kinds of saccharide are contained, one kind may be insoluble saccharide, but the amount thereof is preferably much smaller than the amount of water-soluble saccharide (for example, the insoluble saccharide is about 10 parts by weight or less with respect to 100 parts by weight of the water-soluble saccharide).

In the present specification, a "water-soluble" substance refers to a substance having solubility of 1% by weight or more in water at 37°C. The solubility of the saccharide used in the present invention is preferably about 2% by weight or more, and more preferably about 3% by weight or more, further preferably about 4% by weight or more, and particularly preferably about 5% by weight or more.

Examples of the water-soluble saccharides include: a monosaccharide such as fructose, glucose, and xylose; a disaccharide such as sucrose, maltose, lactose, palatinose, and trehalose; sugar alcohols such as reduced palatinose (also referred to as palatinit), maltitol, sorbitol, erythritol, xylitol, lactitol, mannitol, and reduced starch syrup; decomposed starch such as starch syrup, dextrin, and oligosaccharide; and molasses. Starch syrup and reduced starch syrup include various kinds, and generally, a low-DE type of starch syrup and reduced starch syrup, and a middle-DE type of starch syrup and reduced starch syrup are often used for drop. Here, the low-DE type refers to those having DE of 30 or more and less than 40, and the middle-DE type refers to those having DE of 40 or more and less than 45, and high-DE type refers to those having DE of 45 or more and less than 50. As the DE of them is lower, it can increase the hardness of the candy, and as the DE of them is higher, it can increase the sweetness of the candy. In the production method of the present invention, it is preferable that a reduced starch syrup of a middle-DE type is used.

When sugar is used as the candy base material, it is preferable that a starch syrup is also used. When sugar is used as the candy base material, even in a small amount, a crystal of the sugar exists at the time of endpoint of boiling, seed of recrystallization of the sugar would be remained, which results in promoting return (crystallization) of the product. In the extreme case, crystallization may occur during cooling, but the starch syrup has an effect of suppressing the crystallization.

The saccharide is preferably a saccharide that is not assimilated by oral bacteria. In the present specification, the saccharide that is not assimilated by oral bacteria is referred to as "non-cariogenic saccharide". If the saccharide is assimilated by oral bacteria, taking a tablet containing this saccharide would cause an increase in oral bacteria. Such a saccharide can act negatively against an action of removing tongue coating. Therefore, saccharide is preferably a substance which cannot be assimilated by oral bacteria. Examples of the non-cariogenic saccharide include reduced palatinose, maltitol, reduced starch syrup, xylitol, palatinose, sorbitol, erythritol, trehalose, lactitol, and mannitol. It is preferable that the non-cariogenic saccharide is used as the main component of the candy base material. The non-cariogenic saccharide is preferably selected from the group consisting of reduced palatinose, maltitol, reduced starch syrup, and xylitol, and most preferably a combination of reduced palatinose and reduced starch syrup. It is preferable that the candy base material does not substantially contain sugar. In the case of producing a sugarless candy, the candy base material does not contain sugar.

In the present specification, the term "does not substantially contain" a component means that the content of the component is about 1% by weight or less, preferably about 0.5% by weight or less, more preferably about 0.1% by weight or less, particularly preferably about 0.05% by weight or less, and most preferably about 0.01% by weight or less. Moreover, the reduced palatinose has a characteristic that a non-sticky candy can be produced by using only the reduced palatinose as the excipient.

In the case of producing a sugar candy, the amount of the sugar in the candy base material can be appropriately selected as necessary. In this case, it is preferable that the amount of the sugar occupies about 20% by weight or more, about 30% by weight or more, about 40% by weight or more, about 50% by weight or more, or about 60% by weight or more of weight of the total saccharide (solid content based).

Quality of taste of maltitol is somewhat an "indistinct feeding", compared to sucrose, and maltitol has a different taste which stimulates the throat very slightly. Maltitol is a nonhygroscopic similar extent with sucrose.

A reduced starch syrup is a substance obtained by reducing starch syrup as indicated by its name, and has various types according to the kind of the starch syrup (saccharide composition) to be used the raw material. In general, the reduced starch syrup is divided into two types: high saccharified reduced starch syrup and low saccharified reduced starch syrup. The high saccharified reduced starch syrup has low viscosity and relatively high sweetness, and the low saccharified reduced starch syrup has a high viscosity and relatively low sweetness. A DE (Dextrose Equivalent) of a starch syrup is generally about 35 to 50, and moisture content thereof is about 15 to 20% by weight. DE = (directly reduced saccharide (as glucose)/total solid content) x 100.

Xylitol has an unique sweet taste. Xylitol has an extremely large endothermic effect in dissolving (-37 cal/g) and therefore, the cool feeling is large. Xylitol is evaluated to have better taste than that of erythritol. Xylitol also has a stronger effect of promoting secretion of saliva than that of other sugar alcohols.

As the saccharide, one kind of saccharide may be used alone or a plurality of kinds of saccharide may be used in combination. When a plurality of kinds of saccharide are used, it is preferable that the non-cariogenic saccharide occupies about 20% by weight or more, about 30% by weight or more, about 40% by weight or more, about 50% by weight or more, about 60% by weight or more, about 70% by weight or more, about 80% by weight or more, or about 90% by weight or more of the weight of the total saccharide (solid content based).

In the present specification, a "main component" refers to a component that is preferably contained in an amount of about 5% by weight or more, more preferably about 10% by weight or more, further preferably about 15% by weight or more, and most preferably about 20% by weight or more.

The candy base material can also contain water. Water is utilized for dissolving the main component such as saccharide. Water may be any one of soft water, intermediate water, and hard water. Soft water refers to water having hardness of less than 10°, intermediate water refers to water having hardness of 10° or more and less than 20°, and hard water refers to water having hardness of 20° ormore. Water is preferably soft water or intermediate water, and more preferably soft water. Water can be any water such as tap water, ion-exchanged water, or pure water. Water having few impurities is preferable.

Water is used for dissolving the main component such as saccharide, and almost all of the water is removed by evaporation in boiling. The amount of water to be used is preferably about 1.0% by weight or more, more preferably about 1.5% by weight or more, further preferably about 2.0% by weight or more, further more preferably about 2.5% by weight or more, particularly preferably about 3.0% by weight or more, most preferably about 3.5% by weight or more, of the total of the candy base material. The amount of water to be used is preferably about 25% by weight or less, more preferably about 20% by weight or less, further preferably about 15% by weight or less, further more preferably about 10% by weight or less, particularly preferably about 7.5% by weight or less, and most preferably about 5.0% by weight or less of the total of the candy base material. If the amount of water to be used is too large, the period of time taking until the boiling becomes longer and excessive inversion is occasionally occurred, which occasionally result in excessive coloration. If the amount of water to be used is too small, the saccharide may not be completely dissolved, seed of recrystallization may be remained, and which occasionally result in promoting return (crystallization) of the product. As described above, water may be preliminarily contained in the material used as the candy base material.

When the reduced palatinose and reduced starch syrup are used for the candy base, the amount of the reduced palatinose to be used is preferably about 30% by weight or more, more preferably about 35% by weight or more, further preferably about 40% by weight or more, further more preferably about 45% by weight or more, particularly preferably about 50% by weight or more, and most preferably about 55% by weight or more of the total of the candy base material. The amount of the reduced palatinose to be used is preferably about 85% by weight or less, more preferably about 80% by weight or less, further preferably about 75% by weight or less, further more preferably about 70% by weight or less, particularly preferably about 65% by weight or less, and most preferably about 60% by weight or less of the total of the candy base material. When the reduced palatinose and reduced starch syrup are used for the candy base, the amount of the reduced starch syrup to be used is preferably about 10% by weight or more, more preferably about 15% by weight or more, further preferably about 20% by weight or more, further more preferably about 25% by weight or more, particularly preferably about 30% by weight or more, and most preferably about 35% by weight or more of the total of the candy base material. The amount of the reduced starch syrup to be used is preferably about 65% by weight or less, more preferably about 60% by weight or less, further preferably about 55% by weight or less, further more preferably about 50% by weight or less, particularly preferably about 45% by weight or less, and most preferably about 40% by weight or less of the total of the candy base material.

When another saccharide (particularly, non-cariogenic saccharide or sugar) is used instead of the reduced palatinose and the reduced starch syrup, the invention can be appropriately carried out using these weight ratios.

### (1.3 Minor material)

In addition to the candy base material and the non-thermostable enzyme, various materials can be added for the candy of the present invention.

Examples of the minor material include insoluble materials, nuts, fruits, milk products, table salt, oil and fats, foaming agents, emulsifiers, acidulants, flavors, and colorants.

Examples of the insoluble materials include insoluble dietary fiber (for example, pulp and powdery cellulose), and calcium powder (for example, calcium carbonate powder, calcium phosphate powder, and calcium sulfate powder). If the insoluble material is added into the candy, concavities and convexities are formed on the exposed surface of the candy during the candy is sucked, and therefore, the effect of removing tongue coating, dental plaque, and the like can be exerted. Therefore, it is preferable that the insoluble material is contained in the candy for removing tongue coating and the candy for removing dental plaque.

Examples of the nuts include peanuts, almond, cashew nuts, and pistachio.

Examples of the fruits include citrus, avocado, fig, papaya, kiwi, and pineapple.

Examples of the milk products include condensed milk and sweetened condensed milk. The term "milk product" in the present specification does not include a product that is substantially composed of oil or fat even if the product is derived from milk, and for example, butter is categorized into oil and fat and is not a milk product.

Examples of the oil and fat include shortening, butter, and salad oil liquid oil). The salad oil is useful for improving dispersibility of a powder additive such as an enzyme, and therefore, is occasionally added to the candy base after the powder and the fat are mixed to be in a paste form.

Some minor materials often evaporates and cannot exert the characteristics of these if added at boiling to reduce, and therefore, generally, are added to the candy base material at a cooling stage. The oil and fat are occasionally added for preventing foaming during boiling in producing, for example, ball candy, bekko candy, or the like. In producing molasses peanuts taffy or the like, it is occasional that sugar, molasses, water, and exposed starch syrup are boiled end to 121° C, and then, table salt and peanuts are added thereto, and furthermore, they are heated to 127° C, and then, the residual peanuts are added thereto, and they are cooled down and cut.

The candy is very useful because effective components can be retained in an oral cavity for a long period of time.

The amount of the minor material to be used can be set in the known range in the art according to the kind of the minor material. With respect to 100 parts by weight of the candy base material after moisture evaporation, the total amount of the minor material to be used is preferably about 1 parts by weight or more, more preferably about 2 parts by weight or more, further preferably about 5 parts by weight or more, and most preferably about 10 parts by weight or more. With respect to 100 parts by weight of the candy base material after moisture evaporation, the total amount of the minor material to be used is preferably about 50 parts by weight or less, more preferably about 40 parts by weight or less, further preferably about 30 parts by weight or less, and most preferably about 25 parts by weight or less. If the total amount of the minor material to be used is too small, the effects by adding the minor materials may not be obtained. If the total amount of the minor materials to be used is too large, the obtained candy may not be solidified or it may be difficult to shape the candy.

For example, when the intended candy is a candy for removing tongue coating or a candy for removing dental plaque, with respect to 100 parts by weight of the candy base material after moisture evaporation, the amount of the insoluble material to be used is preferably about 0.1 parts by weight or more, more preferably about 0.2 parts by weight or more, further preferably about 0.5 parts by weight or more, and most preferably about 1 parts by weight or more. With respect to 100 parts byweight of the candy base material after moisture evaporation, the amount of the insoluble material to be used is preferably about 5 parts by weight or less, more preferably about 4 parts by weight or less, further preferably about 3 parts by weight or less, and most preferably about 2 parts by weight or less. If the amount of the insoluble material to be used is too small, the tongue coating removing effect or the dental plaque removing effect is occasionally not obtained. If the amount of the insoluble material to be used is too large, the material occasionally provides inside of the oral cavity with excessive stimulation.

### (2. Method for producing candy)

The production method of the present invention can be performed according to a production method known in the art, except that stirring and temperature control after addition of the non-thermostable enzyme are strictly performed for the purpose of shortening heat history after addition of the non-thermostable enzyme.

In the method for producing a candy, generally, the candy base material is heated to the dissolving temperature, and furthermore, heated from the dissolution temperature to the moisture evaporation temperature, and thereby the moisture is evaporated, and if needed, oil or fat, a milk product, or the like is added and mixed, and the mixture is cooled on the cooling plate. At the time point when the mixture is cooled to a certain temperature, an easily volatile minor material such as perfume and the other minor materials are added and mixed, if needed, and shaped and thereby, the candy is produced.

In the method of the present invention, preferably before cooling with the cooling plate, the non-thermostable enzyme is added at the time point when the temperature of the candy base material lowers by a certain extent, and the material and the enzyme is mixed with a high-speed stirring machine until the non-thermostable enzyme is substantially uniformly dispersed. Addition of the non-thermostable enzyme after cooling with the cooling plate and during cooling with the cooling plate is not preferable because the enzyme will not be uniformly dispersed and the productivity will decrease.

### (2.1 Temperature change of candy base material)

The temperature change of the candy base material in the production method of the present invention will be explained.

First, the candy base material is heated to the dissolution temperature, and the candy base material is heated from the dissolution temperature to moisture evaporation temperature, and the candy base material is cooled from the moisture evaporation temperature to the first cooling temperature, and then, the candy base material is cooled from the first cooling temperature to the second cooling temperature, and then, the candy base material is cooled form the second cooling temperature to the third cooling temperature, and then, the candy base material is cooled from the third cooling temperature to the fourth cooling temperature. These temperature changes are allowed to occur continuously.

The candy base material is not heated from the first cooling temperature to the fourth cooling temperature. Before reaching the first cooling temperature, the heating may be performed after the temperature lowers once.

In the present specification, the term "dissolution temperature" refers to the temperature at which almost all of the candy base material except for water is dissolved in water. The dissolution temperature is preferably about 100° C or higher, more preferably about 101° C or higher, further preferably about 102° C or higher, and further more preferably about 105° C or higher. The dissolution temperature can be, for example, about 110° C or higher, about 115° C or higher, about 120° C or higher, about 125° C or higher, about 130°C or higher, about 135°C or higher, about 136°C or higher, about 137° C or higher, about 138° C or higher, about 139°C or higher, or about 140°C or higher. The dissolution temperature is preferably, about 143° C or lower, more preferably about 142° C or lower, particularly preferably about 141° C or lower, and most preferably 140° C or lower. The dissolution temperature can be, for example, about 135° C or lower, about 130° C or lower, about 125° C or lower, about 120° C or lower, about 115° C or lower, about 110° C or lower, or about 105° C or lower. The dissolution temperature can be appropriately set according to the candy base material to be used.

In the present specification, the term "moisture evaporation temperature" refers to the temperature to be maintained for evaporating water from the candy base material. The moisture evaporation temperature is preferably about 100° C or higher, more preferably about 101° C or higher, further preferably about 102° C or higher, and further more preferably about 105° C or higher. The moisture evaporation temperature can be, for example, about 110° C or higher, about 115° C or higher, about 12 0° C or higher, about 125° C or higher, about 130°C or higher, about 135°C or higher, about 140°C or higher, about 143° C or higher, about 144° C or higher, about 145°C or higher, about 146°C or higher, about 147°C or higher, about 148° C or higher, or about 150° C or higher. The moisture evaporation temperature is preferably about 180° C or lower, more preferably about 170° C or lower, further preferably about 160° C or lower, further more preferably about 155° C or lower, particularly preferably about 153° C or lower, and most preferably about 150°C or lower. The moisture evaporation temperature can be, for example, about 145° C or lower, about 140° C or lower, about 135° C or lower, about 130° C or lower, about 125° C or lower, about 120° C or lower, about 115° C or lower, about 110° C or lower, or about 105°C or lower. The moisture evaporation temperature can be appropriately set according to the candy base material to be used.

In the present specification, the term "first cooling temperature" refers to the temperature at a certain time point after initiation of cooling after moisture was evaporated from the candy base material. For general candies, the first cooling temperature is preferably about 100° C or higher, more preferably about 105° C or higher, further preferably about 110° C or higher, further more preferably about 115° C or higher, particularly preferably about 120° C or higher, and most preferably about 125°C or higher. For general candies, the first cooling temperature is preferably about 135° C or lower, more preferably about 130° C or lower, further preferably about 125° C or lower, further more preferably about 120° C or lower, particularly preferably about 125° C or lower, and most preferably about 120° C or lower. In a certain embodiment (particularly in the case of a sugarless candy), the first cooling temperature is preferably about 125° C or higher, more preferably about 126° C or higher, further preferably about 127° C or higher, further more preferably about 128° C or higher, particularly preferably about 129° C or higher, and most preferably about 130° C or higher. In a certain embodiment (particularly in the case of a sugarless candy), the first cooling temperature is preferably about 135°C or lower, more preferably about 134° C or lower, further preferably about 133° C or lower, further more preferably about 132° C or lower, particularly preferably about 131° C or lower, and most preferably about 130°C or lower. In a certain embodiment (particularly in the case of a sugar candy), the first cooling temperature is preferably about 100° C or higher, more preferably about 101° C or higher, further preferably about 102° C or higher, further more preferably about 103° C or higher, particularly preferably about 104° C or higher, and most preferably about 105° C or higher. In a certain embodiment (particularly in the case of a sugar candy), the first cooling temperature is preferably about 135° C or lower, more preferably about 130° C or lower, further preferably about 125° C or lower, further more preferably about 120° C or lower, particularly preferably about 125° C or lower, and most preferably about 120° C or lower.

In the present specification, the term "second cooling temperature" refers to the temperature at a certain time point when the temperature became lower than the first cooling temperature after initiation of cooling after moisture was evaporated from the candy base material. For a general candies, the second cooling temperature is preferably about 80° C or higher, more preferably about 85° C or higher, further preferably about 90° C or higher, and most preferably about 95° C or higher. For a general candies, the second cooling temperature is preferably about 122° C or lower, more preferably about 121° C or lower, further preferably about 120° C or lower. In a certain embodiment (particularly in the case of a sugarless candy), the second cooling temperature is preferably about 115° C or higher, more preferably about 116° C or higher, and further preferably about 117° C or higher, particularly preferably about 118° C or higher, and most preferably about 119° C or higher. In a certain embodiment (particularly in the case of a sugarless candy), the second cooling temperature is preferably about 122° C or lower, more preferably about 121° C or lower, and furthermore preferably about 120° C or lower. In particular, in the case of a sugarless candy, in one preferable embodiment, the second cooling temperature may be about 119° C or lower, and may most preferably be about 118° C or lower. In a certain embodiment (particularly in the case of a sugar candy), the second cooling temperature is preferably about 80° C or higher, more preferably about 81° C or higher, further preferably about 82° C or higher, further more preferably about 83° C or higher, particularly preferably about 84° C or higher, and most preferably about 85° C or higher. In a certain embodiment (particularly in the case of a sugar candy), the second cooling temperature is preferably about less than 100° C, more preferably about 99° C or lower, further preferably about 98° C or lower, further more preferably about 97° C or lower, particularly preferably about 96° C or lower, and most preferably about 95° C or lower.

In the present specification, the term "third cooling temperature" refers to the temperature at a certain time point when the temperature became lower than the second cooling temperature after initiation of cooling after moisture was evaporated from the candy base material. For general candies, the third cooling temperature is preferably about 50° C or higher, more preferably about 55° C or higher, further preferably about 60° C or higher, further more preferably about 65° C or higher, and most preferably about 70° C or higher. For general candies, the third cooling temperature is preferably about 95° C or lower, more preferably about 93° C or lower, and further preferably about 90° C or lower. In a certain embodiment (particularly in the case of a sugarless candy), the third cooling temperature is preferably about 80°C or higher, more preferably about 81° C or higher, further preferably about 82° C or higher, further more preferably about 83° C or higher, particularly preferably about 84° C or higher, and most preferably about 85°C or higher. In a certain embodiment (particularly in the case of a sugarless candy), the third cooling temperature is preferably about 92° C or lower, more preferably about 91° C or lower, and further more preferably about 90° C or lower. In one embodiment, the third cooling temperature may be about 89°C or lower, about 88°C or lower, or about 87° C or lower. In a certain embodiment (particularly in the case of a sugar candy), the third cooling temperature is preferably about 50° C or higher, more preferably about 51° C or higher, further preferably about 52° C or higher, further more preferably about 53° C or higher, particularly preferably about 54° C or higher, and most preferably about 55° C or higher. In a certain embodiment (particularly in the case of a sugar candy), the third cooling temperature is preferably less than about 80° C, more preferably about 75° C or lower, and further preferably about 70° C or lower.

In the present specification, the term "fourth cooling temperature" refers to the temperature at a certain time point when the temperature became lowered than the third cooling temperature after initiation of cooling after moisture was evaporated from the candy base material. For general candies, the fourth cooling temperature has not a lower limit and is, for example, about 5°C or higher, preferably approximately room temperature (about 15 to 25° C) or more, more preferably about 25°C or higher, further preferably about 30° C or higher, further more preferably about 35° C or higher, particularly preferably about 40° C or higher, and most preferably about 45° C or higher. For general candies, the fourth cooling temperature is preferably less than about 50° C, and in one embodiment, is about 45° C or lower, and may be about 40° C or lower, about 35° C or lower, about 30° C or lower, or about 25° C or lower. In a certain embodiment (particularly in the case of a sugarless candy), the fourth cooling temperature is preferably approximately room temperature (about 15 to 25° C) or more, more preferably about 25° C orhigher, further preferably about 30° C or higher, further more preferably about 35° C or higher, particularly preferably about 40°C or higher, and most preferably about 45°C or higher. In a certain embodiment (particularly in the case of a sugarless candy), the fourth cooling temperature is preferably about 50°C or lower, and in one embodiment, is about 45° C or lower, and may be about 40° C or lower, about 35° C or lower, about 30° C or lower, or about 25° C or lower. In a certain embodiment (particularly in the case of a sugar candy), the fourth cooling temperature is preferably about room temperature (about 15 to 25° C) or more, more preferably about 25° C or higher, further preferably about 30° C or higher, further more preferably about 35° C or higher, particularly preferably about 40° C or higher, and most preferably about 45°C or higher. In a certain embodiment (particularly in the case of a sugar candy), the fourth cooling temperature is preferably about 50°C or lower, and in one embodiment, is about 45° C or lower, and may be also about 40° C or lower, about 35° C or lower, about 30° C or lower, and about 25° C or lower.

The steps of heating a candy base material to a dissolution temperature, heating the candy base material from the dissolution temperature to a moisture evaporation temperature, and cooling the candy base material from the moisture evaporation temperature to a first cooling temperature can be conducted in an appropriate period of time and an appropriate procedure according to a method well-known in the art.

The period of time from the addition of the non-thermostable enzyme to the candy base material until reaching the second cooling temperature is about 5 minutes or less, preferably about 4 minutes and 30 seconds or less, more preferably about 4 minutes or less, and most preferably about 3 minutes and 30 seconds or less.

The period of time from the second cooling temperature to reaching the third cooling temperature is about 5 minutes or less, preferably about 4 minutes and 30 seconds, more preferably about 4 minutes or less, and most preferably about 3 minutes and 30 seconds or less.

The period of time from the third cooling temperature to reaching the fourth cooling temperature is about 20 minutes or less, preferably about 19 minutes or less, more preferably about 18 minutes or less, further more preferably about 17 minutes or less, particularly preferably about 16 minutes or less, and most preferably about 15 minutes or less.

How to perform each step of the processing of the candy during the temperature change of the temperature of the candy base material will be more specifically explained in each of the following items.

### (2.2 Dissolution of candy base material)

The dissolution of a candy base material is performed according to a method known in the art. For example, water, saccharide, and the like are put in a heating vessel (such as a pot) and heated until the saccharide and the like are dissolved with stirring. In the production method of the present invention, the steps from the dissolving step to the candy-obtaining step may be performed with the same batch amount, or for example, a large amount of the candy base material is dissolved at the dissolution step, and the subsequent steps from the moisture evaporation step may be performed with a smaller scale. In order to improve the production efficiency, it is preferable that a large amount of the candy base material is dissolved at the dissolution step and the subsequent steps from the moisture evaporation step are performed with a smaller scale. In this case, a part of the dissolved candy base material that is not yet advanced to the next step is preferably stored by keeping the temperature of the part at constant.

### (2.3 Moisture evaporation from candy base material)

After the dissolution of the candy base material, further heating is performed in order to evaporate moisture from the candy base material. The moisture evaporation from the candy base material is performed according to a method known in the art. The methods for evaporating moisture include a method of boiling until reduced under atmospheric pressure, a method of a vacuum batch-type boiling until reduced, and a method of a continuous vacuum-type boiling until reduced. Any method can be used. In the method of boiling until reduced under atmospheric pressure, for example, a step of boiling to reduce is performed for about 30 to 45 minutes. In the vacuum batch-type boiling until reduced by boiling method, for example, boiling until reduced is performed for about 10 to 20 minutes. In the vacuum continuing type boiling until reduced method, for example, boiling until reduced is performed for about 1-2 minutes.

Composition of the candy base material, temperature for boiling until reduced, and the degree of vacuum at the time of boiling until reduced determine the residual moisture amount of the dough-like mixture of the candy at the endpoint of boiling. The relationships of the temperature for boiling until reduced and the degree of vacuum and the residual moisture amount are known in the art.

For example, when a vacuum chamber of the Super Film Cooker is used to evaporate moisture of the non-sugar candy base material, the pressure can be lowered to about 700 mmHg at about 145° C to evaporate the moisture until the moisture content becomes about 1.5%. For example, when the vacuum chamber of the Super Film Cooker is used to evaporate moisture of the sugar candy base material, the pressure can be lowered to about 700 mmHg at about 140°C to evaporate the moisture until the moisture content becomes about 2.5%.

The amount of the candy base material in performing the moisture evaporation can be any amount. In one embodiment, the moisture evaporation is performed in the batch at the same size as the dissolution step. In another embodiment, the moisture evaporation is performed in the smaller batch than that of the dissolution step. In the case where the batch size at the time of addition of the non-thermostable enzyme or the like after the moisture evaporation is the same as those of the dissolution step, the amount of the candy base material in performing the moisture evaporation is preferably about 15 kg or more, more preferably about 20 kg or more, and most preferably about 22 kg or more. In the case where the batch size at the time of addition of the non-thermostable enzyme or the like after the moisture evaporation is the same as those of the dissolution step, the amount of the candy base material in performing the moisture evaporation is preferably about 35 kg or less, more preferably about 30 kg or less, and most preferably about 27 kg or less.

### (2.4 Addition and mixing of the non-thermostable enzyme and the other minor materials)

After performing the moisture evaporation, the candy base material may be cooled to the first cooling temperature as it is, but in order to enhance production efficiency, it is preferable that the candy base material is transferred onto an apparatus for mixture (such as a kneader).

After the moisture evaporation, the period of time required for cooling the candy base material to the first cooling temperature is an arbitrary period of time. This is because the non-thermostable enzyme is not added at this stage. Therefore, the candy base material may be rapidly cooled by using a forced cooling apparatus or the like, or may be maintained to be a constant temperature, or may be left to stand to cool. The period of time required for cooling from the moisture evaporation temperature to the first cooling temperature is preferably about 5 minutes or less, preferably about 4 minutes and 30 seconds or less, further preferably about 4 minutes or less, and most preferably about 3 minutes and 30 seconds or less. The period of time required for cooling from the moisture evaporation temperature to the first cooling temperature is preferably about 1 minute or more, preferably about 1 minute and 30 seconds or more, further preferably about 2 minutes or more, and most preferably about 2 minutes and 30 seconds or more.

The difference between the moisture evaporation temperature and the first cooling temperature is preferably about 8° C or higher, more preferably about 10° C or higher, further preferably about 15° C or higher, further more preferably about 20° C or higher, particularly preferably about 25° C or higher, and most preferably about 30 or more. The difference between the moisture evaporation temperature and the first cooling temperature is preferably about 65° C or lower, more preferably about 55° C or lower, further preferably about 50° C or lower, further more preferably about 45° C or lower, particularly preferably about 40° C or lower, and most preferably about 35° C or lower.

The candy base material has lower flowability as the temperature lowers although the flowability is very high in a state at a high temperature. When the flowability is lowered to some extent, it is easy to uniformly mix the candy base material. The temperature that is easy to be uniformly mixed is different according to composition of the candy base material. For example, in the case of the sugar candy in which sugar and starch syrup are mainly used as the saccharide, the temperature is about 100 to 110° C. For example, in the sugarless candy in which sugar is not used but the non-cariogenic sugar is used as the saccharide, if the candy base material is mixed at a higher temperature than that of the sugar candy, the candy base material is easy to be uniformly mixed. In the sugarless candy, such a temperature is generally about 120 to 130° C.

Then, while the temperature of the candy base material is between the first cooling temperature (that is, from 100° C or higher to 135°C or lower for general candies, and 125°C to about 135° C for a special case (particularly, in the case of a sugarless candy)) and the second cooling temperature (that is, from 80° C or higher to 122° C or lower for general candies, and 115 to 122° C for a special case (particularly, in the case of a sugarless candy)), the non-thermostable enzyme is added to the candy base material, and the material is stirred so that a substantially uniform mixed dough-like mixture can be obtained within 5 minutes from the addition, and thereby the mixed dough-like mixture is obtained.

The period of time required for cooling the candy base material from the first cooling temperature to the second cooling temperature is an arbitrary period of time. The period of time required before adding the non-thermostable enzyme to the candy base material is not important. Therefore, until the time point before adding the non-thermostable enzyme to the candy base material, the candy base material may be rapidly cooled by using a forced cooling apparatus or the like, or may be maintained at a constant temperature, or may be left to stand to cool.

What is important is the period of time required for cooling to the second cooling temperature from the time point that the non-thermostable enzyme is added to the candy base material. The period of time required for cooling to the second cooling temperature from the time point of addition of the non-thermostable enzyme is preferably about 5 minutes or less, preferably about 4 minutes and 30 seconds or less, further preferably about 4 minutes or less, and most preferably about 3 minutes and 30 seconds or less. The period of time required for cooling to the second cooling temperature from the time of addition of the non-thermostable enzyme is preferably about 1 minute or more, preferably about 1 minute and 30 seconds or more, further preferably about 2 minutes or more, and most preferably about 2 minutes and 30 seconds or more. If the period of time required for cooling to the second cooling temperature from the time of addition of the non-thermostable enzyme is too long, the non-thermostable enzyme is occasionally too inactivated, and if the period of time is too short, the temperature occasionally reach the second cooling temperature before the non-thermostable enzyme is mixed to be substantially uniform.

The candy base material may be rapidly cooled by using a forced cooling apparatus or the like, or the material may be left to stand to cool.

The difference between the first cooling temperature and the second cooling temperature is preferably about 5° C or higher, more preferably about 6° C or higher, further preferably about 7° C or higher, further more preferably about 8° C or higher, particularly preferably about 9° C or higher, and most preferably about 10°C or higher. The difference between the first cooling temperature and the second cooling temperature is preferably about 20°C or lower, more preferably about 17.5° C or lower, further preferably about 15° C or lower, further more preferably about 12.5° C or lower, and most preferably about 10° C or lower.

After the non-thermostable enzyme and the other minor materials as necessary are added, the materials are mixed with stirring. The stirring is performed so that the substantially uniform mixed dough-like mixture can be obtained within about 5 minutes, preferably within about 4 minutes 30 seconds, further preferably within about 4 minutes, most preferably within about 3 minutes 30 seconds from the time of addition of the non-thermostable enzyme. This is performed for the purpose of reducing the heat which is given to the non-thermostable enzyme. The stirring can be performed by using an apparatus known in the art. For example, an apparatus in which a mixing arm (Flavoring Mixer RG) is attached to Mihama MV-350-SFC can be used. This apparatus do not have a forced cooling function, but the temperature of the candy base material lowers naturally during stirring. As the stirring apparatus, an apparatus attached with the forced cooling function may be used.

It is preferable that the stirring is performed at a high speed as much as possible. The stirring may be performed continuously at a high speed, but it is preferable that, on the way, the apparatus is stopped and the candy base material is mixed while the direction of the material is turned or powder attached on the apparatus is scratched, while the level of the dispersion of the non-thermostable enzyme is confirmed. Then, the stirring is performed at a high speed again. If such mixing is performed between these high-speed stirrings, the substantially uniform mixed dough-like mixture can be obtained more rapidly than the case of continuing only to perform the high-speed stirring.

An amount of the candy base material for adding the non-thermostable enzyme can be any amount, but when the capacity of the pot is about 30 liter, the amount can be, for example, about 10 kg or more, and is preferably about 15 kg or more, more preferably about 20 kg or more, and most preferably about 22 kg or more. The amount of the candy base material for adding the non-thermostable enzyme can be any amount, but the amount is preferably about 35 kg or less, more preferably about 30 kg or less, and most preferably about 27 kg or less. If the amount of the candy base material to which the non-thermostable enzyme to be added is too large, the period of time until reaching the second cooling temperature may become too long, and the enzyme is occasionally too inactivated. If the amount of the candy base material to which the non-thermostable enzyme is added is too small, the temperature occasionally reach the second cooling temperature before a substantially uniform mixed dough-like mixture is obtained.

If the amount of the candy base material is in such a range, the weight of the non-thermostable enzyme is preferably about 0.1 kg or more, more preferably about 0.2 kg or more, and most preferably about 0.3 kg or more. The weight of the non-thermostable enzyme can be any amount, but the amount is preferably about 1.0 kg or less, more preferably about 0.8 kg or less, and most preferably about 0. 6 kg or less. If the amount of the non-thermostable enzyme is too large, the temperature occasionally reach the second cooling temperature before the mixed dough-like mixture substantially uniform with candy base material is obtained. If the amount of the non-thermostable enzyme is too small, the intended enzyme activity is occasionally not exerted.

The term "substantially uniform mixed dough-like mixture" in the present specification refers that when about 10 g of dough-like mixtures from any three or more points of one batch of the mixed dough-like mixture are obtained and compared by the naked eye, no difference of dispersion degree of the enzyme is found between each of the dough-like mixtures.

### (2.5 Cooling on cooling plate)

Then, while the temperature of the candy base material is between the second cooling temperature (that is, from 80° C or higher to 122° C or lower for general candies, and 115 to 122° C for a special case (particularly, in the case of a sugarless candy)) and the third cooling temperature (that is, from 50° C or higher to 95° C or lower for general candies, and 80 to 95° C for a special case (particularly, in the case of a sugarless candy)), the mixed dough-like mixture is cooled on a cooling plate. When cooling on the cooling plate, easily volatile minor material such as perfume may be added and mixed.

The period of time required for cooling from the second cooling temperature to the third cooling temperature is preferably about 5 minutes or less, preferably about 4 minutes and 30 seconds or less, further preferably about 4 minutes or less, and most preferably about 3 minutes and 30 seconds or less. The period of time required for cooling from the second cooling temperature to the third cooling temperature is preferably about 1 minute or more, preferably about 1 minute and 30 seconds or more, further preferably about 2 minutes or more, and most preferably about 2 minutes and 30 seconds or more. If the period of time required for cooling from the second cooling temperature to the third cooling temperature is too long, the non-thermostable enzyme is occasionally too inactivated, and if the period of time is too short, the candy base is occasionally solidified uneven.

The candy base material may be rapidly cooled using the cooling plate or the like, or may be left to stand to cool. The candy base material is preferably cooled on the cooling plate.

The difference between the second cooling temperature and the third cooling temperature is preferably about 5°C or higher, more preferably about 6°C or higher, further preferably about 7°C or higher, further more pref erably about 8° C or higher , particularly preferably about 9°C or higher, and most preferably about 10° C or more. The difference between the second cooling temperature and the third cooling temperature is preferably about 20° C or lower, more preferably about 17.5° C or lower, further preferably about 15°C or lower, further more preferably about 12.5° C or lower, and most preferably about 10° C or lower.

The cooling plate is an apparatus having a plate portion for cooling the candy base material. In general, the cooling plate is often produced from metal from the viewpoints of good heat conductivity and cost. The cooling plate can be produced from any metal, and can be made of iron, copper, aluminum, stainless steel, or the like. It is preferable that the cooling plate is made of iron. Water is generally flowed into the cooling plate for the purpose of holding the cooling plate surface in contact with the candy base material to be a low temperature. Water flew into the cooling plate is preferably about 15 to 20° C, and thereby, the cooling plate surface is kept at about 17 to 23° C.

The mixed dough-like mixture is cooled on the cooling plate while being mixed. The mixed dough-like mixture is preferably mixed by a human hand. In performing mixing by a human hand, it is preferable that the person wears heat-resistance gloves. It is preferable that the mixing of the mixed dough-like mixture is performed so that the mixed dough-like mixture is folded. The mixing of the mixed dough-like mixture is by a method known in the art. It is preferable that the mixing is performed well so that the temperature of the total mixed dough-like mixture does not have unevenness.

It is preferable that cooling on the cooling plate is performed so that the period of time from the second cooling temperature (that is, from 80° C or higher to 122° C or lower for general candies, and 115 to 122° C for a special case (particularly, in the case of a sugarless candy)) to the third cooling temperature (that is, from 50° C or higher to 95° C or lower for general candies, and 80 to 95° C for a special case (particularly, in the case of a sugarless candy)) is about 5 minutes or less, preferably about 4 minutes and 30 seconds or less, further preferably 4 minutes or less, and most preferably 3 minutes and 30 seconds or less.

The cooling can be achieved in a short time by cooling with mixing on the cooling plate.

### (2.6 Incorporation of bubbles by kneading)

When the intended candy is a candy for removing tongue coating or a candy for removing dental plaque, if bubbles are incorporated into the mixed dough-like mixture of the candy by kneading, concavities and convexities are formed on the exposed surface of the candy during sucking the candy, and therefore, the effects of removing tongue coating, plaque, and the like can be exerted. Therefore, incorporation of bubbles into the candy for removing tongue coating and the candy for removing dental plaque by needing is preferable.

Incorporation of bubbles into the mixed dough-like mixture of the candy by kneading can be performed according to a method known in the art.

It is preferable that the step of incorporation of bubbles into the mixed dough-like mixture by kneading is performed, after cooling by the cooling plate, while the temperature of the candy base material is between the second cooling temperature and the third cooling temperature.

### (2.7 Shaping)

Then, while the temperature of the candy base material is between the third cooling temperature (that is, from 50° C or higher to 80°C or lower for general candies, and 80 to 95°C for a special case (particularly, in the case of a sugarless candy)) and the fourth cooling temperature (that is, less than 50°C for general candies, and room temperature to 50°C for a special case (particularly, in the case of a sugarless candy)), the mixed dough-like mixture is shaped.

The period of time required for cooling from the third cooling temperature to the fourth cooling temperature is preferably about 20 minutes or less, preferably about 19 minutes or less, further preferably about 18 minutes or less, further more preferably about 17 minutes or less, particularly preferably about 16 minutes or less, and most preferably about 15 minutes or less. The period of time required for cooling from the third cooling temperature to the fourth cooling temperature is preferably about 5 minutes or more, more preferably about 6 minutes or more, further preferably about 7 minutes or more, further more preferably about 8 minutes or more, particularly preferably about 9 minutes or more, and most preferably about 10 minutes or more. If the period of time required for cooling to the third cooling temperature from to the fourth cooling temperature is too long, the non-thermostable enzyme is occasionally too inactivated, and if the period of time is too short, the shaping step is occasionally unsuccessful.

The candy base material may be rapidly cooled by using a cooling conveyer or the like, or may be left to stand to cool. It is preferable that cooling is performed by a cooling conveyer or the like after shaping.

The difference between the third cooling temperature and the fourth cooling temperature is preferably about 5° C or higher, more preferably about 6°C or higher, further preferably about 7° C or higher, further more preferably about 8° C or higher, particularly preferably about 9° C or higher, and most preferably about 10 or higher. The difference between the third cooling temperature and the fourth cooling temperature is preferably about 20°C or lower, more preferably about 17.5° C or lower, further preferably about 15° C or lower, further more preferably about 12.5° C or lower, and most preferably about 10°C or lower.

The mixed dough-like mixture is shaped according to a method known in the art. For example, shaping can be performed by stamp molding or cutting after stretching the mixed dough-like mixture to be slim.

It is preferable that shaping is performed so that the period of time from the third cooling temperature to reaching the fourth cooling temperature is about 20 minutes or less, preferably about 19 minutes or less, further preferably about 18 minutes or less, further more preferably about 17 minutes or less, particularly preferably about 16 minutes or less, and most preferably about 15 minutes or less.

The shape of the candy to be shaped can be any shape.

The shaping of the mixed dough-like mixture can be shaped to have a weight by which the resultant candy exerts the enzyme effect. The weight of the candy can vary with depending on the enzyme content, but the enzyme effect is easier to be exerted as the period of time for the candy is remained in the oral cavity is longer, and therefore, it is preferable that the candy has a weight to some extent. The weight of the candy is preferably about 0.5 g or more, more preferably about 0.75 g or more, and further preferably about 1 g or more. The weight of the candy is preferably about 5 g or less, more preferably about 4 g or less, and further preferably about 3 g or less. If the weight of the candy is too light, the effect of the enzyme is occasionally exerted insufficiently. However, when a number of candies are taken together or with short intervals, the effect of the enzyme can be obtained even when the candies are small. If the weight of the candy is too large, it occasionally becomes difficult to hold the candy in the oral cavity.

As described above, the enzyme-containing candy can be obtained.

### (3. Candy)

The weight of the candy obtained by the production method of the present invention is preferably about 0.5 g or more, more preferably about 0.75 g or more, and further preferably about 1 g or more. The weight of the candy is preferably about 15 g or less, more preferably about 10 g or less, and further preferably about 5 g or less. If the weight of the candy is too light, the effect of the enzyme is occasionally exerted insufficiently. However, when a number of candies are taken together or with short intervals, the effect of the enzyme can be obtained even when the candies are small. If the weight of the candy is too large, it occasionally becomes difficult to hold the candy in the oral cavity.

In the candy obtained by the production method of the present invention, the non-thermostable enzyme is substantially uniformly dispersed.

The enzyme content of the candy obtained by the production method of the present invention is preferably about 0.1% by weight or more, more preferably about 0.2% by weight or more, and further preferably about 0.3% by weight or more. The enzyme content of the candy is preferably about 0.5% by weight or less, more preferably about 0.4% by weight or less, and further preferably about 0.3% by weight or less. If the weight of the candy is too light, the effect of the enzyme is occasionally exerted insufficiently. However, when a number of candies are taken together or with short intervals, the effect of the enzyme can be obtained even when the candies are small. If the weight of the candy is too large, it occasionally becomes difficult to hold the candy in the oral cavity.

### (4. Method for taking the candy)

With respect to the candy obtained according to the production method of the present invention, one candy may be sucked once, or a plurality of (for example, 2 to 10) candies may be sucked at once. When a plurality of candies is sucked at once, a plurality of candies may be put into the mouth at once and sucked, or a plurality of candies may be sequentially sucked one by one.

The uptake frequency of the candy is preferably three times per one day to once per three days, more preferably twice per one day to once per two days, and further preferably once per one day.

The timing of uptake of the candy may be before a meal or after a meal or between meals. The timing can be appropriately determined according to a purpose of taking the candy. For example, if the candy is a candy for removing tongue coating, a lipase-containing candy, a candy for removing dental plaque, or an oxyreductase-containing candy, the uptake after a meal is preferable. Before a meal refers to a period from immediately before a meal to about 30 minutes before a meal, after a meal refers to from immediately after a meal to about 30 minutes after a meal, and between meals refers to from more than about 2 hours after a meal to more than about 2 hours before a subsequent meal.

The uptake period of the candy can be arbitrarily determined. The period is preferably for about one day or more and about one month or less, more preferably for about three days or more and about two weeks or less, and more preferably for about five days or more and about ten days or less. If needed, the candy may be administrated almost permanently.

It is preferable that in the uptake, the candy is held in the oral cavity without swallowing. The candy is held in the oral cavity, preferably for about 10 seconds to about 30 minutes, more preferably for about 1 minute to about 20 minutes, and further preferably for about 3 minutes to about 10 minutes. If the holding time is too short, it is occasionally difficult to obtain the desired effect.

### (5. Method for measuring activities of enzyme contained in the candy)

The activity of the enzyme contained in the candy can be measured by a method known in the art. For measuring the enzyme activity, the candy is crushed and dissolved in an appropriate buffer, and its enzyme activity is measured. In crushing the candy, attention should be paid not to inactivate the enzyme by applying excessive heat to the candy. It is preferable that the temperature of the buffer in dissolving the candy is about 4° C. The methods for measuring activity of various enzymes are known for each enzyme. If the same method is used for the enzyme before adding to the candy and for the enzyme in the candy, any appropriate method can be used. For example, the activity of endoprotease can be measured according to a method that is recommended by the manufacture by using PROTAZYME AK TABLETS manufactured by Megazyme. By this measuring method, activity of all endoproteases active to casein can be measured. Examples of such proteases include papain, proteinase K, trypsin, bovine pancreatic protease, subtilisin A, Bacillus subtilis protease, and Aspergillus niger protease. The measurement may be performed by using a method described in Japanese Standards of Food Additives.

In the candy obtained by the production method of the present invention, it is preferable that about 40% or more of activity of the enzyme used for the production remains, and it is more preferable that about 50% or more remains, and it is further preferable that about 60% or more remains, and it is particularly preferable that about 70% or more remains, and it is the most preferable that about 80% or more remains.

### (6. Method for evaluating an action of removing tongue coating of candy of the present invention)

When the candy of the present invention contains papain, bromelain, or actinidine, the candy of the present invention has an action of removing tongue coating. The phrase "having an action of removing tongue coating" refers to that an action of removing tongue coating is confirmed by the following procedure. Specifically, the dorsum of the tongue of a person who has taken the candy is photographed according to the method described in Evaluation Example 1, and an area of the dorsum of tongue and an area of the tongue coating are obtained, and based thereon, a tongue coating adhesion ratio is calculated. Then, instead of the candy of Example 5, a candy whose action of removing tongue coating is desired to be confirmed (preferably about 1 to about 10 candies, more preferably about 1 to about 5 candies, more preferably about 3 candies) is made to be sucked by the person who has taken the candy without chewing up and without swallowing, similar to Evaluation Example 1. Generally, a candy having the same size and same material as those of Evaluation example 1 takes about 5 to 15 minutes (such as about 10 minutes) for finishing sucking. Immediately after finishing sucking, the dorsum of the tongue is photographed, the area of the dorsum of tongue and the area of tongue coating are measured, and the tongue coating adhesion ratio is calculated based on the areas. If the tongue coating adhesion ratio after the candy uptake is lower than the tongue coating adhesion ratio before the candy uptake, this candy is referred to as a candy having an action of removing tongue coating.

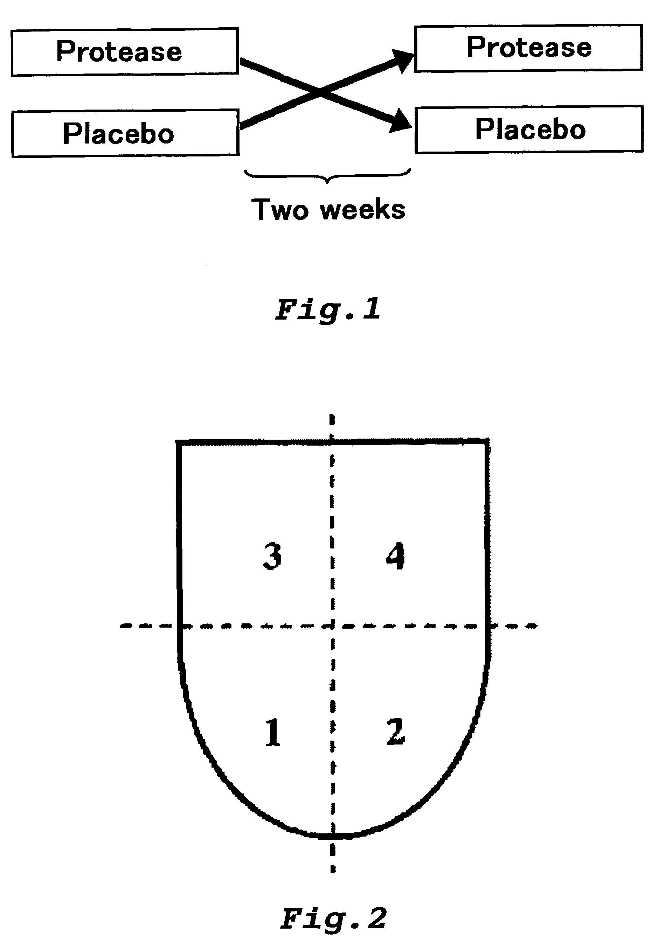

The action of removing tongue coating of the candy of the present invention can also be evaluated by evaluation of tongue coating scores. The tongue coating score is, as explained in Evaluation Example 1, a total of the amount of tongue coating at sites "1" to "4" of the dorsum of the tongue when the amounts of tongue coating adhered to each of the dorsum of the tongue sites "1" to "4" in judgmental rating reference diagram for the amount of tongue coating shown in FIG. 2 are evaluated to each of the sites of the dorsum of the tongue "1" to "4" as (Tongue coating area score) x (Tongue coating thickness score). The maximum tongue coating score is 36 and the minimum tongue coating score is 0. Although a lower tongue coating score is preferable, a state where no tongue coating is present is not preferable (no tongue coating at any of 4 evaluation sites, or a total of tongue coating scores at four locations from 0 to 3 points). It is noted that the normal range free from halitosis is preferably from 4 to 8 points (1 to 2 points for each of the four evaluation sites), and most preferably 4 points (1 point for each of the four evaluation sites). Judgment criteria of the amount of tongue coating at each site is as follows:

**(Table 1)**

| Criteria of scores of tongue coating adhesion area | |
|---|---|
| 0: | No tongue coating |
| 1: | Ratio of tongue coating adhesion area is 1/3 or less |
| 2: | Ratio of tongue coating adhesion area is greater than 1/3 and less than 2/3 |
| 3: | Ratio of tongue coating adhesion area is 2/3 or more |

The criteria of scores of tongue coating thickness are as follows:

**(Table 2)**

| Criteria of scores of tongue coating thickness | |
|---|---|
| 0: | No tongue coating |
| 1: | Lingual papilla are observed |
| 2: | Lingual papilla are almost hidden |
| 3: | Lingual papilla are hidden |

Preferably, tongue coating score after the taking candy is, as compared to tongue coating score before the taking candy, about 1 point or more lower, more preferably about 2 points or more lower, further preferably about 3 points or more lower, even further preferably about 4 points or more lower, even further preferably about 5 points or more lower, even further preferably about 10 points or more lower, and even further preferably about 15 points or more lower.

The candy for removing tongue coating of the present invention can be used for any applications requiring tongue coating removal. For example, the candy is preferably used for humans with tongue coating score of 10 points or more. For example, the candy for removing tongue coating of the present invention can be used for patients with cerebral stroke. Since it is known that in patients with cerebral stroke, pneumonia is induced by excessive accumulation of tongue coating, the candy for removing tongue coating of the present invention can be used for prevention of pneumonia. Since tongue coating is known to be a primary cause for halitosis, the candy for removing tongue coating of the present invention can be used for removal or prevention of halitosis.

As used herein, having "halitosis" refers to that the exhaled breath has an unpleasant odor. In particular, having halitosis refers that causative substances responsible for an unpleasant odor are present in the exhaled breath at the threshold or more. In a more particular case, having halitosis refers that volatile sulfur compounds are present in the exhaled breath in an amount of the threshold or more. In a more particular case, volatile sulfur compound is selected from the group consisting of hydrogen sulfide, methyl mercaptan, and dimethyl sulfide. The threshold at which bad odor is felt when volatile sulfur compound is present in the exhaled breath is 1.5 ng/10 ml of the exhaled breath for hydrogen sulfide, 0.5 ng/10 ml of the exhaled breath for methyl mercaptan, and 0.2 ng/10 ml of the exhaled breath for dimethyl sulfide.

When the halitosis removing treatment is performed to an uptake subject having halitosis, preferably, the treatment is performed so that the amount of at least one of the volatile sulfur compounds in the exhaled breath after taking candy is less than the threshold for feeling bad odor, that is, hydrogen sulfide is less than 1.5 ng/10 ml of the exhaled breath, methyl mercaptan is less 0.5 ng/10 ml of the exhaled breath, or dimethyl sulfide is less than 0.2 ng/10 ml of the exhaled breath. More preferably, the treatment is performed so that all of the amounts of hydrogen sulfide, methyl mercaptan, and dimethyl sulfide are less than the threshold for feeling bad odor.

Preferably, the amount of any volatile sulfur compounds in the exhaled breath after candy digestion is lower than the amount of the volatile sulfur compound in the exhaled breath before taking the candy by about 5% or more, more preferably lower by about 10% or more, more preferably lower by about 15% or more, further more preferably lower by about 20% or more, further more preferably lower by about 25% or more, further more preferably lower by about 30% or more, further more preferably lower by about 45% or more, and further more preferably lower by about 50% or more.

Although detailed mechanisms are not understood, a candy having an action of removing tongue coating has preferably an action of reducing production of volatile sulfur compounds by oral bacteria. Therefore, when the subject takes this candy, generation of halitosis after uptake can be prevented for a certain period of time in addition to removing halitosis already present at the time of uptake. Accordingly, the candy of the present invention can be used for both of removal and prevention of halitosis.

The candy of the present invention can be used for any applications which require halitosis removal or prevention. For example, the candy of the present invention is preferably used for humans with volatile sulfur compounds at the threshold (that is, 1.5 ng/10 ml of the exhaled breath for hydrogen sulfide, 0.5 ng/10 ml of the exhaled breath for methyl mercaptan, or 0.2 ng/10 ml of the exhaled breath for dimethyl sulfide) or more. It is considered that the candy of the present invention reduces production of volatile sulfur compounds due to oral bacteria by acting primarily on tongue coating.

### (7. Method of using the candy of the present invention)

The amount of uptake, uptake frequency, uptake period, and the like of the tablet of the present invention can be determined based on the preference of the uptake subject, similar to general candies. When the candy of the present invention contains papain, bromelain, or actinidine and is used for the purpose of removing tongue coating or halitosis, the uptake amount, uptake frequency, and uptake period of the candy of the present invention are determined depending on the conditions of the uptake subject, tongue coating scores of the uptake subject, and the like.

The amount of uptake of the candy of the present invention is preferably about 0.1 g or more, more preferably about 0.2 g or more, further more preferably about 0.5 g or more, and further more preferably about 1 g or more per uptake. Although the amount of uptake of the candy of the present invention has no particular upper limit, it is, for example, about 1000 g or less, about 750 g or less, about 500 g or less, about 250 g or less, about 100 g or less, about 50 g or less, about 40 g or less, about 30 g or less, about 20 g or less, about 10 g or less, about 7.5 g or less, about 5 g or less, about 4 g or less, about 3 g or less, about 2 g or less, about 1 g or less or the like per uptake.

The uptake frequency of the candy of the present invention can be set arbitrarily. For example, the uptake frequency can be once or more a week, twice or more a week, three or more times a week, four or more times a week, five or more times a week, six or more times a week, seven or more times a week, once or more a day, twice or more a day, three or more times a day or the like. The uptake frequency of the candy of the present invention has no upper limit, and can be, for example, three times or less a day, twice or less a day, once or less a day, seven or less times a week, six or less times a week, five or less times a week, four or less times a week, three or less times a week, twice or less a week, once or less a week, or the like.

Although the timing of uptake of the candy of the present invention may be before a meal, after a meal or between meals, after a meal is preferable. Before a meal refers to a period from immediately before a meal to about 30 minutes before a meal, after a meal refers to from immediately after a meal to about 30 minutes after a meal, and between meals refers to from more than about 2 hours after a meal to more than about 2 hours before a subsequent meal.

The uptake period of the candy of the present invention can be determined arbitrarily. The candy of the present invention can be taken preferably for about 1 day or more, more preferably for about 3 days or more, and most preferably for about 5 days or more. The candy of the present invention can be taken preferably for about 1 month or less, more preferably for about 2 weeks or less, and most preferably for about 10 days or less. When necessary, the candy of the present invention may be taken substantially permanently.

At the time of uptake, the candy of the present invention is preferably retained in the oral cavity without swallowing. The period of time for retention of the candy of the present invention in the oral cavity is preferably for about 10 seconds or more, more preferably for about 1 minute or more, and further preferably for about 3 minutes or more. The period of time for retention of the candy of the present invention in the oral cavity is preferably for about 30 minutes or less, more preferably for about 20 minutes or less, and further preferably for about 10 minutes or less. When retention time is too short, it is difficult to attain tongue coating removal effects.

The candy of the present invention is preferably sucked without biting till the end.

If the candy of the present invention contains papain, bromelain, or actinidine and is used for the purpose of removing the tongue coating or halitosis, with the candy of the present invention, an effect of improving taste sensitivity can be expected in addition to an effect of improving the hygienic status of the oral cavity due to removal of tongue coating. These effects are due to the fact that foods can be easily contacted with the lingual papilla after the removal of tongue coating which covers the surface of the tongue.

### EXAMPLES

### (Examples 1-3: Production of papain-containing sugarless candy)

Papain-containing sugarless candies were produced. The outline will be explained. First, candy base materials were dissolved in a large pot, and moisture was removed in a vacuum state, and additives were rapidly mixed in a small pot, and the mixture was cooled on a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140°C and mixed.

The candy base materials were completely dissolved and sufficiently mixed to uniform, and then the candy base materials were temporally stored in a stock tank.

The total amount of the candy base materials was transferred to a heating can of the Super Film Cooker, and then the temperature of the candy base materials was adjusted to 145°C.

The total amount of the candy base materials was placed in a vacuum chamber of the Super Film Cooker and the pressure was decreased to 700 mmHg at 145° C, and the moisture was removed to moisture content of 1.5% by weight. When the removal of moisture in the candy base materials is finished, it can be said that it is in a condition where the candy base material is boiled up.

In an apparatus in which a mixing arm (Flavoring Mixer RG) was attached to a kneader with a capacity of about 30 liters (Mihama MV-350-SFC), 25 kg of the above-described candy base material after the moisture evaporation was placed and stirred. When the temperature of the candy base material became 130° C, a mixture of 0.4kg of citric anhydride (powder), 0.03 kg of a high intensity sweetener (powder) and papain (0.25 kg (Example 1), 0.50 kg (Example 2) or 0.75 kg (Example 3); powder; manufactured by Amano Enzyme Inc.), and 0.1 kg of an orange flavor (liquid) were added thereto and stirred for one minute. Then, the rotation was stopped. While confirming the dispersion of the powders, a mixing step was performed for 1.5 minutes with human hands during which the direction of the candy base material was changed with human hands and the powder attached to the wall of the vessel was detached. Then, a stirring step was performed for one minute to obtain a mixed dough-like mixture. The temperature of the mixed dough-like mixture at the time point of completion of the stirring step was 120°C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 130°C to 120°C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

The mixed dough- like mixture of a temperature of about 90° C was flowed into batch rolls and stretched to be slim.

The stretched mixed dough-like mixture (the temperature was about 90° C) was passed through sizing rolls so that it is stretched to be further slimer.

Then, the slim stretched dough-like mixture (the temperature was about 80°C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40°C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 4 g. The moisture content of the sugarless candy was 1.5% by weight.

### (Measurement Example 1: Measurement of enzyme activity of papain-containing sugarless candy)

The papain-containing sugarless candies produced in Examples 1 to 3 were hermetically-sealed in an airtight pouch and tightened by a vise and thereby crashed to be in powder state. Each of the powder candies was dissolved at 4°C in Buffer B described in an instruction of PROTAZYME AK TABLETS to obtain a solution. The obtained solution was appropriately diluted, and the papain activity was measured by using PROTAZYME AK TABLETS according to the method described in the instruction thereof. As a result, when 0.25 kg of papain (1 part by weight of papain based on 100 parts by weight of the candy base material) was added (Example 1), the ratio of the remaining activity was 70%. When 0.50 kg of papain (2 parts by weight of papain based on 100 parts by weight of the candy base material) was added (Example 2), the ratio of the remaining activity was 75%. When 0.75 kg of papain (3 parts by weight of papain based on 100 parts by weight of the candy base material) was added (Example 3), the ratio of the remaining activity was 80%.

As a result, as a larger amount of the enzyme was added, the remaining activity of the enzyme was higher. It can be thought that since the application of heat is relatively smaller as the enzyme concentration is higher, the enzyme activity becomes easy to remain.

### (Examples 4 to 5 and Comparative Example 1: Production of enzyme-containing candy)

Candies containing bromelain or actinidine were produced. As a Comparative Example, candies containing no enzyme were also produced. The outline will be explained. First, the candy base materials were dissolved in a large pot, and moisture was removed in atmospheric pressure state, and in a small pot, additives were rapidly mixed with them, and the materials were cooled on a cooling iron plate and shaped to obtain sugarless candies.

Specifically, 324 kg of sugar, 216 kg of reduced starch syrup (DE40) and 100 kg of water were placed in a tank for dissolving the raw materials, and heated to 150° C and mixed. The candy base materials were completely dissolved and sufficiently mixed to uniform, and then the candy base materials were temporally stored in a stock tank.

The total amount of the candy base material was transferred to a kneader, and then, with keeping the candy base material at 150° C, the moisture was removed under atmospheric pressure until the moisture content became 1.5% by weight.

In an apparatus in which a mixing arm (Flavoring Mixer RG) was attached to a kneader with a capacity of about 30 liters (Mihama MV-350-SFC), 25 kg of the above-described candy base materials after the moisture evaporation was placed and stirred. When the temperature of the candy base material became 135° C, 0.25 kg of citric anhydride (powder), 0.1 kg of an apple flavor (liquid), and 0.01 kg of a mint flavor (powder) (Comparative Example 1); or a mixture of 0.25 kg of citric anhydride (powder) and 0.25 kg of bromelain (powder; manufactured by Amano Enzyme Inc.), as well as 0.1 kg of an apple flavor (liquid), and 0.01 kg of a mint flavor (powder) (Example 4); or a mixture of 0.25 kg of citric anhydride (powder) and 0.03 kg of bromelain (powder; manufactured by Amano Enzyme Inc.) and 0.03 kg of actinidine (powder; manufactured by Asahi Food and Healthcare Co. , Ltd.), as well as 0.1 kg of an apple flavor (liquid), and 0.01 kg of a mint flavor (powder) (Example 5) were added thereto and stirred for one minute. Then, the rotation was stopped. While confirming the dispersion of the powders, a mixing was performed for 1.5 minutes with human hands during which the direction of the candy base material was changed with human hands and the powder attached to the wall of the vessel was detached. Then, a stirring step was performed for one minute to obtain a mixed dough-like mixture. The temperature of the mixed dough-like mixture at the time point of completion of the stirring step was 120°C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 130°C to 120° C was about 4 minutes.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched dough-like mixture (the temperature was about 90° C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended candies. The shape of the obtained candies was elliptic cylinder. The weight of the obtained candy was about 3.5 g. The moisture content of the candy was 1.5% by weight.

The ratio of the remaining enzyme activity of the candy was measured according to the method described in Measurement Example 1. As a result, when 0.25 kg of bromelain (1 part by weight of bromelain based on 100 parts by weight of the candy base material) was added (Example 4), the ratio of the remaining activity was 65%. When 0.03 kg of bromelain and 0.03 kg of actinidine (0.12 parts by weight of each of the substances based on 100 parts by weight of the candy base material) were added (Example 5), the ratio of the remaining activity was 39%. When an enzyme was not added (Comparative Example 1), the enzyme activity was not detected.

### (Examples 6 to 7: Investigation of temperature at the time of enzyme addition in the production of enzyme-containing candy)

Candies containing bromelain and actinidine were produced. The outline will be explained. First, the candy base material was dissolved in a large pot, and moisture was removed under atmospheric pressure state, and in a small pot, additives were rapidly mixed with them, and the materials were cooled on a cooling iron plate and shaped to obtain candies.

Specifically, 324 kg of sugar, 216 kg of reduced starch syrup (DE40) and 100 kg of water were placed in a tank for dissolving the raw materials, and heated to 150° C and mixed. The candy base materials were completely dissolved and sufficiently mixed to uniform, and then the candy base materials were temporally stored in a stock tank.

The total amount of the candy base material was transferred to a kneader, and then, with keeping the candy base material at 150°C, the moisture was removed under atmospheric pressure until the moisture content became 1.5% by weight.

In an apparatus in which a mixing arm (Flavoring Mixer RG) was attached to a kneader with a capacity of about 30 liters (Mihama MV-350-SFC), 25 kg of the above-described candy base material after the moisture evaporation was placed and stirred. When the temperature of the candy base material became 115°C (Example 6) or 100°C (Example 7), a mixture of 0.25 kg of citric anhydride (powder) and 0.03 kg of bromelain (powder; manufactured by Amano Enzyme Inc.) and 0.03 kg of actinidine (powder; manufactured by Asahi Food and Healthcare Co., Ltd.), and 0.1 kg of an apple flavor (liquid), and 0.01 kg of a mint flavor (powder) were added and stirred for one minute. Then, the rotation was stopped. While confirming the dispersion of the powders, a mixing step was performed for 1.5 minutes with human hands during which the direction of the candy base material was changed with human hands and the powder attached to the wall of the vessel was detached. Then, a stirring step was performed for one minute to obtain a mixed dough-like mixture. The temperature of the mixed dough-like mixture at the time point of completion of the stirring step was 105°C (Example 6) or 95°C (Example 7). In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered to 105° C from 115° C (Example 6) or to 95° C from 100° C (Example 7) was about 3.5 minutes.

The mixed dough-like mixture was mixed on a cooling iron plate with human hands (wearing heat-resistant glove) while the mixed dough-like mixture was folded. The mixture was cooled to 90° C. The cooling time for lowering the temperature to 90°C from 105°C (Example 6) or 95° C (Example 7) was about 4 minutes. The iron plate surface temperature of the cooling iron plate was maintained at between 17 to 23°C by cooling water flowing in the iron plate.

The mixed dough-likemixture of a temperature of about 90° C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80°C) was passed through a stamping apparatus and thereby stamp-molded with a candy mold to obtain candies having a temperature of about 80° C. During the roll-shaping step and the stamp-molding step, neither particular forced heating nor particular forced cooling operation was performed. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended candies. The shape of the obtained candies was elliptic cylinder. The weight of the obtained candy was about 3.5 g. The moisture content of the candy was 1.5% by weight.

The ratio of the remaining enzyme activity of the candy was measured according to the method described in Measurement Example 1. As a result, when the temperature at the time of the enzyme addition was 115°C (Example 6), the ratio of the remaining activity was 40%. When the temperature at the time of the enzyme addition was 100°C (Example 7), the ratio of the remaining activity was 50%.

### (Example 8: Production of enzyme-containing sugarless candy)

Sugarless candies containing bromelain and actinidine were produced. The outline will be explained. First, the candy base materials were dissolved in a large pot, and moisture was removed in a vacuum state, and in a small pot, additives were rapidly mixed with them, and the materials were cooled on a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140° C and mixed. The candy base materials were completely dissolved and sufficiently mixed to uniform, and then, the candy base material was temporally stored in a stock tank.

The total amount of the candy base material was transferred to a heating can of a Super Film Cooker, and then the temperature of the candy base materials was adjusted to 145°C. The total amount of the candy base material was placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base material after the moisture evaporation was placed and stirred. When the temperature of the candy base material became 130° C, a mixture of 0.4 kg of citric anhydride (powder), 0.075 kg of a high intensity sweetener (powder), and 0.03 kg of bromelain (powder; manufactured by Amano Enzyme Inc.) and 0.03 kg of actinidine (powder; manufactured by Asahi Food and Healthcare Co. , Ltd.), as well as 0.1 kg of an apple flavor (liquid), and 0.01 kg of a mint flavor (powder) were added and stirred for one minute, and then, the rotation was stopped. While confirming the dispersion of the powders, a mixing step was performed for 1. 5 minutes with human hands during which the direction of the candy base material was changed with human hands and the powder attached to the wall of the vessel was detached. Then, a stirring step was performed for one minute to obtain a mixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 130° C to 120° C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C (Example 6) or 95° C (Example 7) was about 3.5 minutes. The iron plate surface temperature of the cooling iron plate was maintained at between 17 to 23°C by cooling water flowing in the iron plate.

Themixeddough-likemixture of a temperature of about 90° C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80°C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 4 minutes. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was elliptic cylinder. The weight of the obtained sugarless candy was about 4 g. The moisture content of the sugarless candy was 1.5% by weight.

The ratio of the remaining enzyme activity of the candy was measured according to the method described in Measurement Example 1. As a result, the ratio of the remaining activity was 37%.

### (Example 9: Production of enzyme-containing sugarless candy)

Candies containing bromelain was produced. The outline will be explained. First, the candy base material was dissolved in a large pot, and moisture was removed in atmospheric pressure state, and in a small pot, additives were rapidly mixed with them, and the materials were cooled on a cooling iron plate and shaped to obtain sugarless candies.

Specifically, 613 kg of 70% maltitol syrup was placed in a tank for dissolving the raw materials, and heated to 140° C and mixed. The candy base materials were temporally stored in a stock tank.

The total amount of the candy base materials was transferred to a kneader, and then, with holding the temperature of the candy base materials to be 140°C, the moisture was removed under atmospheric pressure to 2.0% by weight of moisture content.

In an apparatus in which a mixing arm (Flavoring Mixer RG) was attached to a kneader with a capacity of about 30 liters (Mihama MV-350-SFC), 10 kg of the above-described candy base materials after the moisture evaporation were placed and stirred, and when the temperature of the candy base materials became 120° C, 0.25 kg of bromelain (powder; manufactured by Amano Enzyme Inc.), 0.1 kg of an orange flavor (liquid), and 0.01 kg of a mint flavor (powder) were added and stirred for 2 minutes, and then, the rotation was stopped. While confirming the dispersion of the powders, a mixing step was performed for 1.5 minutes with human hands during which the direction of the candy base material was changed with human hands and the powder attached to the wall of the vessel was detached. Then, a stirring step was performed for one minute to obtain a mixed dough-like mixture. The temperature of the mixed dough-like mixture at the time point of the completion of the stirring stepcompletion of the stirring step was 110°C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 120° C to 110° C was about 3 minutes.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 110° C was about 3 minutes. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched dough-like mixture (the temperature was about 90° C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80° C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 5 minutes to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 2 g. The moisture content of the sugarless candy was 2.0% by weight.

The ratio of the remaining enzyme activity of the candy was measured according to the method described in Measurement Example 1. As a result, the ratio of the remaining activity was 60%.

### (Example 10: Production of enzyme-containing candy)

Candies containing papain were produced. The outline will be explained. First, the candy base materials were dissolved in a large pot, and moisture was removed in a vacuum state, and in a small pot, additives were rapidly mixed with them, and the materials were cooled on a cooling iron plate and shaped to obtain candies.

Specifically, 150 kg of reduced palatinose, 150 kg of sugar, 200 kg of starch syrup (DE40), and 100 kg of water were placed in a tank for dissolving the raw materials, and heated to 145°C and mixed. The candy base materials were completely dissolved and sufficiently mixed to uniform, and then, the candy base materials were temporally stored in a stock tank.

The total amount of the candy base material was transferred to a heating can of a Super Film Cooker, and then the temperature of the candy base materials was adjusted to 160°C. The total amount of the candy base material was placed in a vacuum chamber of a Super Film Cooker and the pressure was decreased to 700 mmHg at 160° C, and the moisture was removed to moisture content of 1.5% by weight.

In an apparatus in which a mixing arm (Flavoring Mixer RG) was attached to a kneader with a capacity of about 30 liters (Mihama MV-350-SFC), 20 kg of the above-described candy base material after the moisture evaporation was placed and stirred. When the temperature of the candy base material became 125°C, the mixture of 0.1 kg of citric anhydride (powder) and 0.05 kg of papain (powder; manufactured by Amano Enzyme Inc.), as well as 0.1 kg of an orange flavor (liquid), and 0.05 kg of an apple flavor (liquid) were added and stirred for one minute and 30 seconds, and then, the rotation was stopped. While confirming the dispersion of the powders, a mixing step was performed for 1. 5 minutes with human hands during which the direction of the candy base material was changed with human hands and the powder attached to the wall of the vessel was detached. Then, a stirring step was performed for 2 minutes to obtain a mixed dough-like mixture. The temperature of the mixed dough-like mixture at the time point of the completion of the stirring step was 100° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 125° C to 100° C was about 3 minutes and 30 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 100° C was about 3 minutes and 30 seconds. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched dough-like mixture (the temperature was about 90° C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 10 minutes for the temperature to reach 40°C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended candies. The shape of the obtained candies was cylinder. The weight of the obtained candy was about 3 g. The moisture content of the candy was 1.2% by weight.

The ratio of the remaining enzyme activity of the candy was measured according to the method described in Measurement Example 1. As a result, the ratio of the remaining activity was 65%.

### (Comparative Example 2: Test of production of enzyme-containing candy according to conventional production method)

Candies containing actinidine were produced according to a conventional method. The outline will be explained. First, the candy base materials were dissolved in a large pot, and moisture was removed in a vacuum state, and in a small pot, additives were rapidly mixed with them, and the material was cooled on a cooling iron plate and shaped to obtain sugarless candies.

Specifically, 300 kg of reduced palatinose, 150 kg of reduced starch syrup (DE40), and 20 kg of water were placed in a tank for dissolving the raw materials, and heated to 160° C and mixed. The candy base materials were completely dissolved and sufficiently mixed to uniform, and then, the candy base materials were temporally stored in a stock tank.

The total amount of the candy base material was transferred to a heating can of a Super Film Cooker, and then the candy base material was adjusted to 150° C. The total amount of the candy base material was placed in a vacuum chamber of a Super Film Cooker and the pressure was decreased to 700 mmHg at 150° C, and the moisture was removed to moisture content of 1.0% by weight.

In a kneader with a capacity of about 30 liters (Mihama MV-350-SFC), 30 kg of the above-described candy base materials after the moisture evaporation were placed and stirred by a single arm, and when the temperature of the candy base materials became 140° C, a mixture of actinidine (0.03 kg; powder, manufactured by Asahi Food and Healthcare Co., Ltd.) was added thereto and stirred for 10 minutes, and then, the rotation was stopped. While confirming the dispersion of the powders, a mixing step was performed for 0.5 minutes with human hands during which the direction of the candy base materials was changed with human hands and the powder attached to the wall of the vessel was detached. Then, a stirring step was performed for 10 minutes to obtain a mixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base material lowered from 140° C to 120° C was about 25 minutes.

The mixed dough-like mixture was mixed on a cooling iron plate with human hands (wearing heat-resistant glove) while the mixed dough-like mixture was folded. The mixture was cooled to 90° C. The cooling time for lowering the temperature to 90°C from 120°C was about 10 minutes. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched dough-like mixture (the temperature was about 90° C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 15 minutes to reach 40° C after the shaping.

The cooled candies were subjected to an apparatus for selection against defect candies, and thereby, the candies having a nonstandard sizewere automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was globular. The weight of the obtained sugarless candy was about 5 g. The moisture content of the sugar less candy was 1.0% by weight.

The ratio of the remaining enzyme activity of the candy was measured according to the method described in Measurement Example 1. As a result, the ratio of the remaining activity was 0%.

### (Example 11: Production of lipase enzyme-containing candy)

Sugarless candies containing lipase were produced. The outline will be explained. First, the candy base materials were dissolved in a large pot, and moisture was removed in a vacuum state, and in a small pot, additives were rapidly mixed with them, and the materials was cooled by a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140° C and mixed. The candy base materials were completely dissolved and sufficiently mixed to uniform, and then, the candy base materials were temporally stored in a stock tank.

The total amount of the candy base materials was transferred to a heating can of the Super Film Cooker, and then the temperature of the candy base materials was adjusted to 145°C. The total amount of the candy base material was placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base material after the moisture evaporation was placed and stirred. When the temperature of the candy base material became 130° C, a mixture of 0. 4 kg of citric anhydride (powder), 0.03 kg of a high intensity sweetener (powder), and 0.50 kg of lipase (powder; lipase AY manufactured by Amano Enzyme Inc.), as well as 0.1 kg of an orange flavor (liquid) were added and stirred for one minute, and then, the rotation was stopped. While confirming the dispersion of the powders, a mixing step was performed for 1.5 minutes with human hands during which the direction of the candy base materials was changed with human hands and the powder attached to the wall of the vessel was detached. Then, a stirring step was performed for one minute to obtain a mixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base materials lowered from 130° C to 120° C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

The mixed dough-like mixture of a temperature of about 90°C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80°C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 4 g. The moisture content of the sugarless candy was 1.5% by weight.

The produced ligase-containing sugarless candies were hermetically-sealed in an airtight pouch and tightened by a vise and thereby crashed to be in powder state. The lipase activity of the powder candy was measured by using Lipase Kit S (manufactured by Dainippon Pharmaceutical Co., Ltd.). The remaining ratio was 30% or more.

### (Example 12: Production of amylase-containing candy)

Sugarless candies containing amylase were produced. The outline will be explained. First, the candy base materials were dissolved in a large pot, and moisture was removed in a vacuum state, and in a small pot, additives were rapidly mixed with them, and the materials were cooled by a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140° C and mixed. The candy base materials were completely dissolved and sufficiently mixed to uniform, and then, the candy base materials were temporally stored in a stock tank.

The total amount of the candy base materials was transferred to a heating can of the Super Film Cooker, and then the temperature of the candy base materials was adjusted to 145° C. The total amount of the candy base material was placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base materials after the moisture evaporation was placed and stirred. When the temperature of the candy base materials became 130° C, a mixture of 0.4 kg of citric anhydride (powder), 0.03 kg of a high intensity sweetener (powder), and 0.5 kg of amylase (powder; amylase AD manufactured by Amano Enzyme Inc.), as well as 0.1 kg of an orange flavor (liquid) were added and stirred for one minute, and then, the rotation was stopped. While confirming the dispersion of the powders, a mixing step was performed for 1.5 minutes with human hands during which the direction of the candy base materials was changed with human hands and the powder attached to the wall of the vessel was detached. Then, a stirring step was performed for one minute to obtain a mixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base materials lowered from 130° C to 120° C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

Themixeddough-likemixture of a temperature of about 90° C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80°C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 4 g. The moisture content of the sugarless candy was 1.5% by weight.

The produced amylase-containing sugarless candies were hermetically-sealed in an airtight pouch and tightened by a vise and thereby crashed to be in powder state. The remaining ratio was 30% or more when the amylase activity of the powder candy was measured by using alpha-amylase measuring kit (manufactured by Biocon (Japan) Ltd.).

### (Example 13: Production of enzyme-containing xylitol candy)

A xylitol candy containing papain was produced. The outline will be explained. First, the candy base material was dissolved in a large pot, and moisture was removed in a vacuum state, and in a small pot, additives were rapidly mixed with them, and the materials were cooled by a cooling iron plate and shaped to obtain sugarless candies.

Specifically, 270 kg of reduced palatinose, 30 kg of xylitol, 185 kg of reduced starch syrup (DE40), and 100 kg of water were placed in a tank for dissolving the raw materials, and heated to 140°C and mixed. The candy base materials were completely dissolved and sufficiently mixed to uniform, and then, the candy base material was temporally stored in a stock tank.

The total amount of the candy base materials was transferred to a heating can of the Super Film Cooker, and then the temperature of the candy base materials was adjusted to 145° C. The total amount of the candy base materials was placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base materials after the moisture evaporation was placed and stirred. When the temperature of the candy base materials became 130° C, a mixture of 0.4 kg of citric anhydride (powder), 0.03 kg of a high intensity sweetener (powder), and 0.25 kg of papain (powder; manufactured by Amano Enzyme Inc.) as well as 0.1 kg of an orange flavor (liquid) were added and stirred for one minute, and then, the rotation was stopped. While confirming the dispersion of the powders, a mixing step was performed for 1.5 minutes with human hands during which the direction of the candy base materials was changed with human hands and the powder attached to the wall of the vessel was detached. Then, a stirring step was performed for one minute to obtain amixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base materials lowered from 130° C to 120° C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80°C) was passed through a stamping apparatus and thereby stamp-molded with a candy mold to obtain candies having a temperature of about 80° C. During the roll-shaping step and the stamp-molding step, neither particular forced heating nor particular forced cooling operation was performed. The period of time from the time point of the temperature of 90°C to the completion of the shaping step was 5 minutes. The shaped candies were cooled to 40°C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended xylitol candies. The shape of the obtained xylitol candies was square pole that has a thickness of 8 mm, a longitudinal length of 19 mm, and a horizontal length of 19 mm and that is concave in the central portion. The weight of the obtained xylitol candy was about 4 g. The moisture content of the xylitol candy was 1.5% by weight.

The ratio of the remaining enzyme activity of the xylitol candy containing papain was measured according to the method described in Measurement Example 1. As a result, the ratio of the remaining activity was 70%.

### (Example 14: Production of candy containing enzyme and calcium carbonate)

A sugarless candy containing papain and calcium carbonate was produced. The outline will be explained. First, the candy base materials were dissolved in a large pot, and moisture was removed in a vacuum state, and in a small pot, additives were rapidly mixed with them, and the material was cooled on a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140° C and mixed. The candy base materials were completely dissolved and sufficiently mixed to uniform, and then, the candy base materials were temporally stored in a stock tank.

The total amount of the candy base materials was transferred to a heating can of the Super Film Cooker, and then the temperature of the candy base materials was adjusted to 145° C. The total amount of the candy base materials were placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base materials after the moisture evaporation was placed and stirred. When the temperature of the candy base materials became 130° C, amixture of 0.4 kg of citric anhydride (powder), 0.03 kg of a high intensity sweetener (powder), 0.5 kg of calcium carbonate (powder), and 0.5 kg of papain (powder; manufactured by Amano Enzyme Inc.) as well as 0.1 kg of an orange flavor (liquid) were added and stirred for one minute, and then, the rotation was stopped. While confirming the dispersion of the powders, a mixing step was performed for 1.5 minutes with human hands during which the direction of the candy base materials was changed with human hands and the powder attached to the wall of the vessel was detached. Then, a stirring step was performed for one minute to obtain a mixed dough-like mixture. The temperature of the mixed dough-like mixture at the time point of the completion of the stirring stepcompletion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base materials lowered from 130°C to 120°C was about 3 minutes and 40 seconds.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23° C by cooling water flowing in the iron plate.

The mixed dough-like mixture of a temperature of about 90° C was flowed into batch rolls and stretched to be slim. The stretched mixed dough-like mixture (the temperature was about 90° C) was passed through sizing rolls so that it is stretched to be further slimer. Then, the slim stretched dough-like mixture (the temperature was about 80° C) was passed through a stamping apparatus and thereby stamp-molded with a candy mold to obtain candies having a temperature of about 80° C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40°C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 4 g. The moisture content of the sugarless candy was 1.5% by weight.

The ratio of the remaining enzyme activity of the produced papain-containing candy was measured according to the method described in Measurement Example 1. As a result, the ratio of the remaining activity was 75%.

### (Example 15: Production of enzyme-containing bubble candy)

Sugarless candies containing bromelain were produced. The outline will be explained. First, the candy base materials were dissolved in a large pot, and moisture was removed in a vacuum state, and in a small pot, additives were rapidly mixed with them, and the materials were cooled on a cooling iron plate and shaped to obtain sugarless candies.

Specifically, 300 kg of reduced palatinose, 185 kg of reduced starch syrup (DE40), and 100 kg of water were placed in a tank for dissolving the raw materials, and heated to 140° C and mixed. The candy base materials were completely dissolved and sufficiently mixed to uniform, and then, the candy base materials were temporally stored in a stock tank.

The total amount of the candy base materials was transferred to a heating can of the Super Film Cooker, and then the temperature of the candy base materials was adjusted to 145° C. The total amount of the candy base materials was placed in a vacuum chamber of the Super Film Cooker and the pressure was decreased to 700 mmHg at 145° C, and the moisture was removed to moisture content of 1.5% by weight.

In an apparatus in which a mixing arm (Flavoring Mixer RG) was attached to a kneader with a capacity of about 30 liters (Mihama MV-350-SFC), 25 kg of the above-described candy base materials after the moisture evaporation was placed and stirred. When the temperature of the candy base material became 130° C, the mixture of 0.4 kg of citric anhydride (powder), 0.03 kg of a high intensity sweetener (powder), and 0.03 kg of bromelain (powder; manufactured by Amano Enzyme Inc.) as well as 0.1 kg of an orange flavor (liquid) were added and stirred for one minute, and then, the rotation was stopped. While confirming the dispersion of the powders, a mixing step was performed for 1.5 minutes with human hands during which the direction of the candy base materials was changed with human hands and the powder attached to the wall of the vessel was detached. Then, a stirring step was performed for one minute to obtain a mixed dough-like mixture. The temperature of the mixed dough-like mixture at the time point of the completion of the stirring step was 120° C. In the mixed dough-like mixture at the time point of the completion of the stirring step, the enzyme powder was substantially uniformly mixed. During the steps of addition and stirring, neither particular forced heating nor particular forced cooling was performed. The period of time that takes when the temperature of the candy base materials lowered from 130° C to 120° C was about 3 minutes and 40 seconds.

Bubbles were incorporated into candies by pulling candies for one minute using vertical type candy-pulling machine (manufactured by Mihama Corporation). During this step, there was no substantial lowering of the temperature of the mixed dough-like mixture.

The mixed dough-like mixture was mixed on a cooling iron plate with human hands (wearing heat-resistant glove) while the mixed dough-like mixture was folded. The mixture was cooled to 90° C. The cooling time for lowering the temperature to 90° C from 120° C was about 4 minutes. The iron plate surface temperature of the cooling iron plate was maintained at between 17 to 23°C by cooling water flowing in the iron plate.

The mixed dough- like mixture of a temperature of about 90° C was flowed into batch rolls and stretched to be slim. The stretched mixed dough-like mixture (the temperature was about 90°C) was passed through sizing rolls so that it is stretched to be further slimer. Then, the slim stretched dough-like mixture (the temperature was about 80°C) was passed through a stamping apparatus and thereby stamp-molded with a candy mold to obtain candies having a temperature of about 80° C. During the roll-shaping step and the stamp-molding step, neither particular forced heating nor particular forced cooling operation was performed. The period of time from the time point of the temperature of 90° C to the completion of the shaping step was 5 minutes. The shaped candies were cooled to 40° C through a cooling tunnel. It took 10 minutes for the temperature to reach 40° C after the shaping.

The cooled candies were subjected to an apparatus for selection against defect candies, and thereby, the candies having a nonstandard size were automatically excluded. Then, the candies having break, crack, or poor mixing state were excluded with human hands. The proportion of the excluded candies was less than about 1% of the produced candies. The remaining candies were the intended sugarless candies. The shape of the obtained sugarless candies was square pole that has a thickness of 8 mm, a longitudinal length of 19 mm, and a horizontal length of 19 mm and that is concave in the central portion. The weight of the obtained sugarless candy was about 3.9 g. The moisture content of the sugarless candy was 1.5% by weight.

The ratio of the remaining enzyme activity of the bromelain containing candy was measured according to the method described in Measurement Example 1. As a result, the ratio of the remaining activity was 38%.

### (Summary of Examples 1 to 15 and Comparative Examples 1 and 2)

The materials of Examples 1 to 15 and Comparative Examples 1 and 2 are summarized in the following Table 3. The temperature conditions and results of Examples 1 to 15 and Comparative Examples 1 and 2 are summarized in the following Table 4.

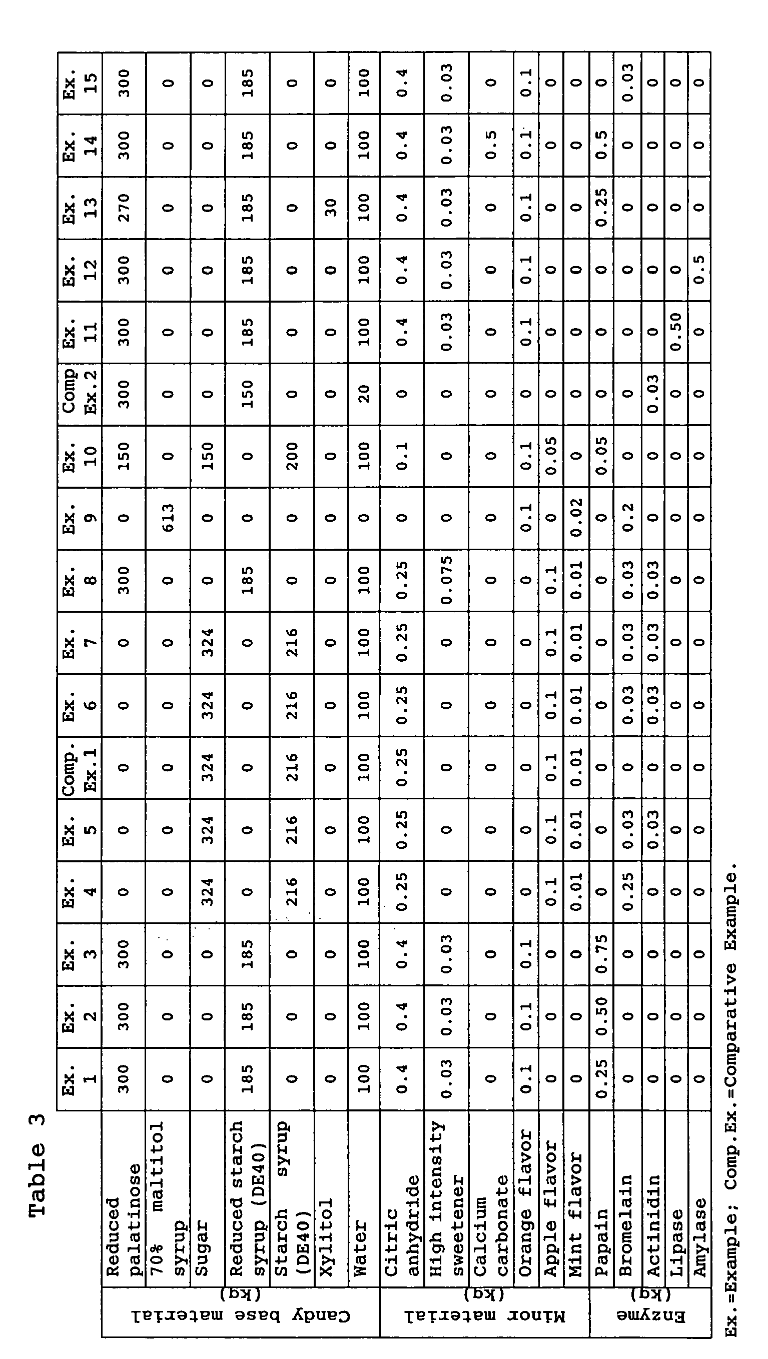

**Table 4**

| | Ex. 1 | Ex. 2 | Ex. 3 | Ex. 4 | Ex. 5 | Comp .Ex. 1 | Ex. 6 | Ex. 7 | Ex. 8 | Ex. 9 | Ex. 10 | Comp .Ex. 2 | Ex. 11 | Ex. 12 | Ex. 13 | Ex. 14 | Ex. 15 |
|---|---|---|---|---|---|---|---|---|---|---|---|---|---|---|---|---|---|
| Dissolution temperature (°C) | 140 | 140 | 140 | 150 | 150 | 150 | 150 | 150 | 140 | 140 | 145 | 160 | 140 | 140 | 140 | 140 | 140 |
| Moisture evaporation temperature (°C) | 145 | 145 | 145 | 150 | 150 | 150 | 150 | 150 | 145 | 140 | 160 | 150 | 145 | 145 | 145 | 145 | 145 |
| Moisture evaporation conditions | Vac. | Vac. | Vac. | At | At | At | At | At | Vac. | At | Vac. | Vac. | Vac. | Vac. | Vac. | Vac. | Vac. |
| Enzyme addition temperature (°C) | 130 | 130 | 130 | 135 | 135 | 135 | 115 | 100 | 130 | 120 | 125 | 140 | 130 | 130 | 130 | 130 | 130 |
| Candy base upon addition of enzyme (kg) | 25 | 25 | 25 | 25 | 25 | 25 | 25 | 25 | 25 | 10 | 20 | 30 | 25 | 25 | 25 | 25 | 25 |
| Stirring time (min) | 1 | 1 | 1 | 1 | 1 | 1 | 1 | 1 | 1 | 2 | 1.5 | 10 | 1 | 1 | 1 | 1 | 1 |
| Cooling time from beginning of cooling on cooling iron plate to 90° C (min) | 4 | 4 | 4 | 4 | 4 | 4 | 4 | 4 | 4 | 3 | 3.5 | 10 | 4 | 4 | 4 | 4 | 4 |
| Weight of candy (g) | 4 | 4 | 4 | 3.5 | 3.5 | 3.5 | 3.5 | 3.5 | 4 | 2 | 3 | 5 | 4 | 4 | 4 | 4 | 3.9 |
| Shape of candy | S.p | S.p | S.p | Oval | Oval | Oval | Oval | Oval | Oval | S.p | Circ le | Glob ular | S.p | S.p | S.p | S.p | S.p |
| Cooling time to 40°C after shaping (min.) | 10 | 10 | 10 | 10 | 10 | 10 | 10 | 10 | 10 | 5 | 10 | 15 | 10 | 10 | 10 | 10 | 10 |
| Moisture content (%) of finished product | 1.5 | 1.5 | 1.5 | 1.5 | 1.5 | 1.5 | 1.5 | 1.5 | 1.5 | 2.0 | 1.2 | 1.0 | 1.5 | 1.5 | 1.5 | 1.5 | 1.5 |
| Ratio of remaining Enzyme activity (%) | 70 | 75 | 80 | 65 | 39 | 0 | 40 | 50 | 37 | 60 | 65 | 0 | ≧30 | ≧30 | 70 | 75 | 38 |

| | | | | | | | | | | | | | | | | | |
|---|---|---|---|---|---|---|---|---|---|---|---|---|---|---|---|---|---|
| Ex.=Example; Comp.Ex.=Comparative Example. Vac.=Vacuum; At=Atmospheric pressure; S.p=Saquare pole | | | | | | | | | | | | | | | | | |

### (Evaluation Example 1: Confirmation of tongue coating removal effect and halitosis suppression effect of protease-containing candy)

### (1. Test subject, study design, and procedure)

Eight adult men whose consents of participation of the evaluation study were obtained and who had tongue coatings and volatile sulfur compounds (hereinafter, abbreviated as VSC) are detected in gases in their oral cavities were used as test subjects.

Prohibition of tongue cleaning from three days before the examination to the day of the examination and prohibition of oral cavity activities (such as oral cavity cleaning, eating and drinking, and smoking) from uprising of the day of the examination to the completion of the examination were prescribed to the test subjects.

As shown in Fig. 1, the design of the evaluation study was a double-blind cross matching test, and washout period was set to be two weeks.

Each of the test subjects was made to uptake one candy to be evaluated (candy of Example 5 or Comparative Example 1), and before and after the uptake, the amounts of the tongue coating adhesion were evaluated. In taking the candy, the candy was taken with slowly sucked and dissolved mainly on the dorsum of tongue without chewed. It took about 8 minutes to finish sucking the candy.

Immediately before the uptake of the candy and immediately after the uptake, the surface of the dorsum of tongue was photographed and a tongue coating adhesion ratio was evaluated by visual inspection. Immediately before the uptake of the candy and 90 minutes after the uptake, gas in their oral cavity was sampled, and the VSC concentration was measured.

### (2. Evaluation method of tongue coating amount) (1) Photography method

The dorsum of tongue of the test subject was photographed by using a digital camera (FinePix S602, Fujifilm Corporation) attached a Ring Flash (14RDX, YUZO). By this method, a photograph without shadow is photographed. The color chart for correcting color tone (CasMatch, Dai Nippon Printing Co. , Ltd.) was disposed alongside the photographic subject and photographed, and by using the chart, brightness of the photograph was corrected.

### (2) Evaluation method by visual inspection

Evaluation of an amount of tongue coating by visual inspection was performed by modifying the method described in Moriya et al. (MORIYA Toshiki, KISHI Mitsuo, AIZAWA Fumie, et al., "Zettai Score Ni Yoru Koshu Screening No Yukosei Ni Kansuru Kenkyu (Tongue Coating Score as a Screening Test for Oral Malodor)", Journal of Dental Health 52: 12-21, 2002) by taking the thickness of the tongue coating into consideration. That is, the photographed dorsum of tongue was divided into four areas, and the tongue coating adhesion amount of each of the areas was evaluated as (tongue coating adhesion area score) x (tongue coating thickness score), the total thereof was considered as the tongue coating score, and thereby the evaluation was performed. The evaluation was based on an average of the values of three persons who observed the tongue coating. The tongue coating adhesion area scores and the tongue coating thickness scores were respectively ranked as one of the four stages of 0 to 3.

1) Dorsum of tongue is divided into four areas;
2) Tongue coating adhesion amount is evaluated by an area and a thickness of tongue coating adhered to each of the areas:
   (tongue coating adhesion area score) x (tongue coating thickness score); and
3) The total of the scores of the respective areas is the tongue coating score.

The criteria of the tongue coating adhesion area score were as follows:

**(Table 5)**

| Criteria of scores of tongue coating adhesion area | |
|---|---|
| 0: | No tongue coating |
| 1: | Ratio of tongue coating adhesion area is 1/3 or less |
| 2: | Ratio of tongue coating adhesion area is greater than 1/3 and less than 2/3 |
| 3: | Ratio of tongue coating adhesion area is 2/3 or more |

The criteria of scores of tongue coating thickness are as follows:

**(Table 6)**

| Criteria of scores of tongue coating thickness | |
|---|---|
| 0: | No tongue coating |
| 1: | Lingual papilla are observed |
| 2: | Lingual papilla are almost hidden |
| 3: | Lingual papilla are hidden |

### (3. Evaluation method of halitosis)

As VSC, H₂S and CH₃SH were focused on. This is because contribution of H₂S and CH₃SH to halitosis is high. Concentrations of H₂S and CH₃SH were measured by using Oral Chroma (ABILIT Corporation). A test subject was prescribed to breathe through the nose with taking 10 ml-syringe in the mouth with keeping quiet for 30 seconds, after one deep breath. Then, 10 ml of the oral gas was sampled, and 5 ml thereof was injected into Oral Chroma (ABILIT Corporation). By special analysis software of a personal computer connected to the Oral Chroma, concentrations of H₂S and CH₃SH were calculated as weights (ng/10 ml) in 10 ml of the oral gas. The collection of the sample and the measurement of concentrations of H₂S and CH₃SH were initiated between 9 a.m. and 9:30 a.m. through the examination and performed by the same measuring person.

### (4. Statistical analysis)

The results obtained for the tongue coating score were subjected to t-test for the tongue coating score immediately after using the candy assuming that the tongue coating score immediately before using the candy was 100%. When P is 0.05 or less, it was considered that there is a significant difference. For the statistical treatment, SPSS 10.0J for Windows (registered trademark) (SPSS Inc., USA) was used.

The results obtained for VSC were subjected to t-test for the relative concentrations of H₂S and CH₃SH 90 minutes after using the candy considering the concentrations of H₂S and CH₃SH immediately before using a candy were 100% respectively. P < 0.05 or less was considered ad there is a significant difference. For the statistical treatment, SPSS 10.0J for Windows (registered trademark) (SPSS Inc., USA) was used.

### (5. Results)

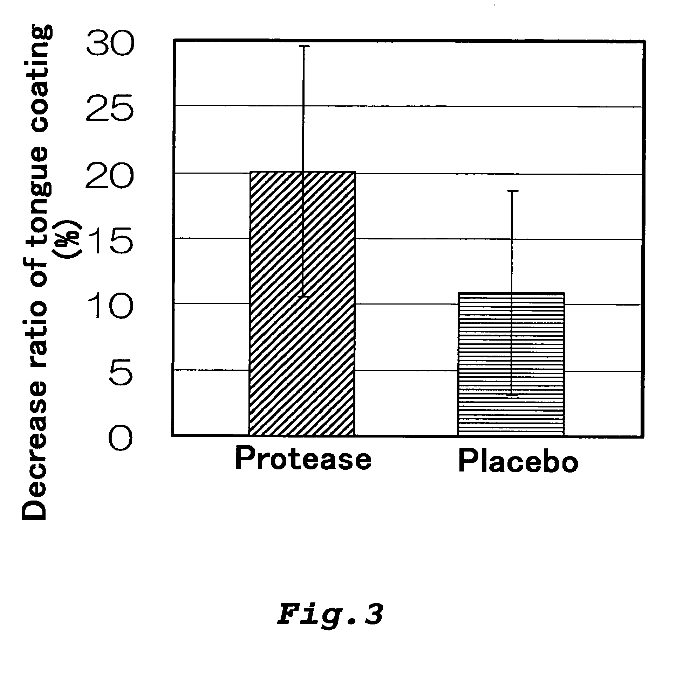

The results relating to the tongue coating removing effect are shown in Fig. 3. In both of the protease group and the placebo group, as compared to before use, the tongue coating score decreased significantly after use. The decrease ratio was about 20% in the protease group, and about 10% in the placebo group.

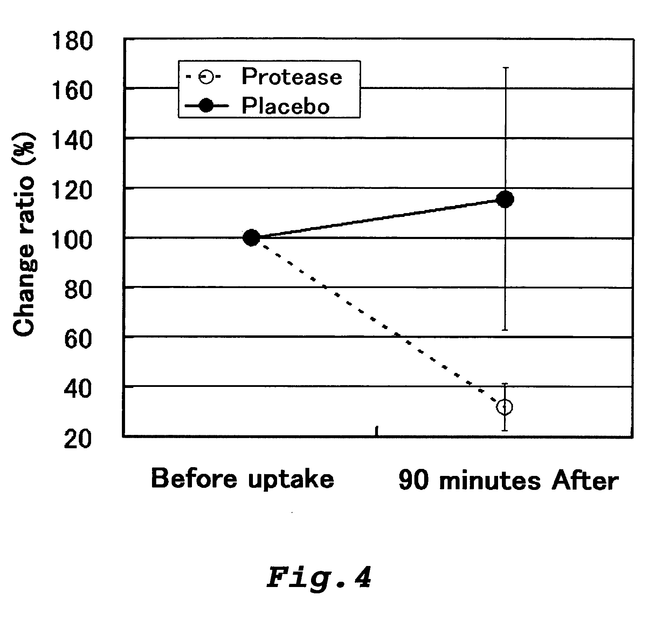

The results for the VSC suppressing effect are shown in Fig. 4. Even after 90 minutes, the protease group significantly maintained a lower value as compared with that before use. On the other hand, the placebo group had no significant difference after 90 minutes of use.

As mentioned above, the present invention is exemplified as mentioned above using preferable embodiments of the present invention. However, these embodiments should not be construed as limiting the scope of the present invention. It is understood that the scope of the present invention will be interpreted by the appended claims. Those skilled in the art will understand that from detailed preferable embodiments of the present invention, equivalent scope can be carried out based on the description of the present invention and common technical practice. It is understood that patents, patent applications and publications cited in the present specification are herein incorporated by reference for the content thereof as if the contents themselves were specifically described in the present specification.

### INDUSTRIAL APPLICABILITY

According to the present invention, a candy having an enzyme activity is provided. Preferably, in the candy of the present invention, the enzyme is almost uniformly distributed.

## Claims

1. A method for producing an enzyme-containing candy, in which
a candy base material is heated to a dissolution temperature,
the candy base material is heated from the dissolution temperature to a moisture evaporation temperature,
the candy base material is cooled from the moisture evaporation temperature to a first cooling temperature, then
the candy base material is cooled from the first cooling temperature to a second cooling temperature, then
the candy base material is cooled from the second cooling temperature to a third cooling temperature, and then
the candy base material is cooled from the third cooling temperature to a fourth cooling temperature,
wherein there is no substantial increase of the temperature of the material from the first cooling temperature to the fourth cooling temperature,
the dissolution temperature is 100° C or higher and 143° C or lower,
the moisture evaporation temperature is 100° C or higher and 180°C or lower,
the first cooling temperature is 100°C or higher and 135°C or lower,
the second cooling temperature is 80°C or higher and 122°C or lower,
the third cooling temperature is 50° C or higher and 95° C or lower, and
the fourth cooling temperature is less than 50°C, and wherein
the method comprising the steps of:
(a) adding an non-thermostable enzyme to the candy base material, stirring the material so as to obtain a substantially uniform mixed dough-like mixture within 5 minutes from the addition, and thereby obtaining the mixed dough-like mixture;
(b) cooling the mixed dough-like mixture on a cooling plate; and
(c) shaping the mixed dough-like mixture;
wherein step (a) is performed while the temperature of the candy base material is between the first cooling temperature and the second cooling temperature;
the step (b) is performed while the temperature of the candy base material is between the second cooling temperature and the third cooling temperature;
the step (c) is performed while the temperature of the candy base material is between the third cooling temperature and the fourth cooling temperature;
the period of time from the addition of the non-thermostable enzyme to the candy base material until reaching the second cooling temperature is 5 minutes or less;
the period of time from the second cooling temperature to reaching the third cooling temperature is 5 minutes or less; and
the period of time from the third cooling temperature to reaching the fourth cooling temperature is 20 minutes or less.

2. The method according to claim 1, wherein a main component of the candy base material is a saccharide.

3. The method according to claim 2, wherein the main component is a non-cariogenic saccharide.

4. The method according to claim 2, wherein the main component is selected from the group consisting of reduced palatinose, maltitol, reduced starch syrup, and xylitol.

5. The method according to claim 2, wherein the main component is reduced palatinose.

6. The method according to claim 1, wherein the candy base material does not substantially contain sugar.

7. The method according to claim 1, wherein the temperature is 80°C or lower at which a remaining activity is 20% or less after the non-thermostable enzyme is heated for 30 minutes in a 20 mM Tris buffer (pH 7.0).

8. The method according to claim 1, wherein the non-thermostable enzyme is selected from the group consisting of a transferase, hydrolase, lyase, isomerase, and ligase.

9. The method according to claim 1, wherein the non-thermostable enzyme is selected from the group consisting of a protease, lipase, amylase, and dextranase.

10. The method according to claim 1, wherein the non-thermostable enzyme is selected from the group consisting of a cysteine protease and serine protease.

11. The method according to claim 1, wherein the non-thermostable enzyme is selected from the group consisting of a papain, bromelain, and actinidine.

12. The method according to claim 1, wherein the non-thermostable enzyme is added in a powder state.

13. The method according to claim 1, wherein the weight of the candy base material in step (a) is from 20 to 30 kg.

14. The method according to claim 13, wherein the weight of the non-thermostable enzyme in step (a) is from 0.2 to 1 kg.

15. The method according to claim 1, wherein the non-thermostable enzyme is substantially uniformly dispersed in the enzyme-containing candy.

16. The method according to claim 1, wherein the enzyme content of the enzyme-containing candy is from 0.1 to 3.0% by weight.

17. The method according to claim 1, wherein the enzyme-containing candy is a candy for removing tongue coating, the enzyme is a protease, and the candy base material contains an insoluble powder.

18. The method according to claim 1, wherein the enzyme-containing candy is a candy for removing tongue coating, and the enzyme is a protease, and wherein the method further comprises step (d) of kneading bubble in the mixed dough-like mixture, and step (d) is performed while the temperature of the candy base material is between the second cooling temperature and the third cooling temperature after step (b).
